# EUROPEAN PATENT SPECIFICATION

(11) **EP 2 158 268 B1**
(45) Date of publication and mention of the grant of the patent: **09.01.2013**
(21) Application number: 08756483.7
(22) Date of filing: 30.05.2008
(51) Int. Cl.: C08L 83/14, C08L 83/04, C08G 77/50

(54) **SILICONE COPOLYMERS AND ELASTOMERS DERIVED FROM NATURAL OILS**
VON NATÜRLICHEN ÖLEN ABGELEITETE SILIKONCOPOLYMERE UND ELASTOMERE
COPOLYMÈRE ET ÉLASTOMÈRE DE SILICONE DÉRIVÉS D'HUILES NATURELLES

(30) Priority: 30.05.2007 US 940769 P; 29.05.2008 US 57031 P
(43) Date of publication of application: 03.03.2010
(73) Proprietor: Dow Corning Corporation, Midland, Michigan 48686-0994 (US); Dow Corning do Brasil Limitada, CEP 05686-010 Sao Paulo (BR)
(72) Inventor: DIAS, Tania, Cristina, 01425-011 Sao Paulo (BR); ALMEIDA, Daniel, Ferreira, 13033-590 Campinas, Sao Paulo (BR); LIN, Shaow, Midland, MI 48642 (US)
(74) Representative: Polypatent
(86) International application number: PCT/US2008/065202
(87) International publication number: WO 2008/150947

(56) References cited:
- US-A1- 2005 027 051

## Description

### Technical Field

This invention relates to compositions containing the reaction products of an organohydrogenpolysiloxane, a natural oil, and a hydrosilylation catalyst, as well as methods for preparing such compositions, and their use in personal care products.

### Background

Silicones and vegetable oils (also referred to as natural oils) are common ingredients in many personal care formulations, each providing unique attributes or performance benefits to the final formulation. There has been a long standing interest to identify new ingredients for personal care formulations, often using the approach of preparing new molecules by combining structural features from different classes of compounds into one. This approach has led to the development of numerous organic modified silicones that are commonplace in many of the commercial personal care products sold today. To this end, there has been an interest to combine silicones with vegetable oils to produce personal care ingredients retaining the unique attributes of each class of materials, while possibly discovering new or better attributes. However, there have been few, if any, successful attempts to prepare vegetable oils chemically modified through the addition of a silicone.

The document US 2005/027051 A1 (O'BRIEN MICHAEL J [US] ET AL) 3 February 2005 (2005-02-03) shows a cosmetic composition comprising a hydrogen polysiloxane, vegetable oil as an inert dispersant and a hydrosilylation catalyst.

The present inventors have discovered a process to modify vegetable oils with silicones. The process involves hydrosilylating the unsaturated carbon-carbon bonds present in vegetable oils with an organohydrogenpolysiloxane. While hydrosilylation reactions are well known in the art, they have not been used to successfully prepare silicone modified vegetable oils.

The present inventors have surprisingly discovered that hydrosilylation of the carbon-carbon double bonds in vegetable oils with an organohydrogenpolysiloxane is possible and the process can be used to produce a variety of new compounds considered to be silicone-vegetable oil copolymers. The reaction may be used to prepare silicone-vegetable oil copolymers having elastomeric properties. The elastomeric silicone-vegetable oil copolymers may be blended with various silicone and organic low molecular weight fluids to form gel compositions.

### Summary

This invention relates to a composition comprising the reaction products of;
A) an organohydrogenpolysiloxane having at least two silicon bonded hydrogen atoms,
B) a vegetable oil containing at least one aliphatic unsaturated bond,
C) a hydrosilylation catalyst, and
D) an optional crosslinking compound containing at least two terminal aliphatic unsaturated groups in its molecule,
F) an optional alpha olefin compound,
wherein the reaction products contain at least one silicone copolymer formed by the addition of the silicon bonded hydrogen atoms of the organohydrogensiloxane across the aliphatic unsaturated bond in the vegetable oil.

In one embodiment, the silicone copolymer comprises an organopolysiloxane containing at least one structural unit of the average formula

R¹ₘ (E)SiO _{(3-m)/2}

where;
R¹ is a monovalent hydrocarbon group,
E is a fatty ester group attached to silicon in the structural unit by at least one
Si - C bond wherein the carbon atom of the bond is one of the carbon
atoms originally present in the aliphatic unsaturated bond of the vegetable oil, m may vary from 0 to 2.

The present invention also relates to a process for preparing a silicone modified vegetable oil comprising reacting;
A) an organohydrogenpolysiloxane having at least two SiH units,
B) a vegetable oil containing at least one aliphatic unsaturated bond, and
C) a hydrosilylation catalyst,
in the presence of E) an optional solvent.

In one embodiment, a process is disclosed for preparing a silicone copolymer composition comprising:
I) reacting
   A) an organohydrogenpolysiloxane having an average formula

      [(CH₃)HSiO]_{g}

      where g is 3 to 8;
   B) a vegetable oil containing at least one aliphatic unsaturated bond; and
   C) a hydrosilylation catalyst;
      wherein the reaction product still contains some un-reacted SiH units,
II) further reacting the product of step I) with
   D) a crosslinking compound containing at least two terminal aliphatic unsaturated groups in its molecule in the presence of
   E) an optional solvent.

The present invention further relates to personal care product compositions containing the silicone copolymers as disclosed herein.

### Detailed Description

The silicone copolymers and elastomers of the present disclosure contain at least one Si-C based reaction product of an organohydrogenpolysiloxane, a vegetable oil, and a hydrosilylation catalyst. The silicone copolymers and elastomers are obtainable via a hydrosilylation reaction between the organohydrogenpolysiloxane and the vegetable oil. The hydrosilylation reaction may be conducted neat or in the presence of a solvent. The silicone modified vegetable oils may be obtained by the process of the present invention comprising reacting;
A) an organohydrogenpolysiloxane having at least two silicon bonded hydrogen atoms,
B) a vegetable oil containing at least one aliphatic unsaturated bond,
C) a hydrosilylation catalyst, and
D) an optional crosslinking compound containing at least two terminal aliphatic unsaturated groups in its molecule,
F) an optional alpha olefin compound.

### Components A - F are described below.

### A) The Organohydrogenpolysiloxane

Component A) of the present invention is an organohydrogenpolysiloxane having an average, per molecule, of at least two silicon bonded hydrogen atoms. As used herein, an organohydrogenpolysiloxane is any organopolysiloxane containing a silicon-bonded hydrogen atom (SiH). Organopolysiloxanes are polymers containing siloxane units independently selected from (R₃SiO_{0.5}), (R₂SiO), (RSiO_{1.5}), or (SiO₂) siloxy units, where R may be any monovalent organic group. When R is a methyl group in the (R₃SiO_{0.5}), (R₂SiO), (RSiO_{1.5}), or (SiO₂) siloxy units of an organopolysiloxane, the siloxy units are commonly referred to as M, D, T, and Q units respectively. These siloxy units can be combined in various manners to form cyclic, linear, or branched structures. The chemical and physical properties of the resulting polymeric structures can vary. For example organopolysiloxanes can be volatile or low viscosity fluids, high viscosity fluids/gums, elastomers or rubbers, and resins. Organohydrogenpolysiloxanes are organopolysiloxanes having at least one SiH containing siloxy unit. When the SiH unit is present on a M, D, or T siloxy unit, the siloxy units may be represented as comprising of "M^{H}" siloxy units (R₂HSiO_{0.5}), "D^{H}" siloxy units (RHSiO), "T^{H}" siloxy units (HSiO_{1.5}). Thus, the organohydrogenpolysiloxanes useful in the present invention may comprise any number of M, M^{H}, D, D^{H}, T, T^{H}, or Q siloxy units, providing at least one siloxy unit contains SiH, and there is on average at least two SiH units in the molecule. Component (A) can be a single organohydrogenpolysiloxane or a combination comprising two or more organohydrogenpolysiloxanes that differ in at least one of the following properties; structure, viscosity, average molecular weight, siloxane units, and sequence.

In one embodiment, the organohydrogenpolysiloxane comprises a linear, SiH terminated, organohydrogenpolysiloxane of general formula HR¹₂SiO-[R¹₂SiO]ₓ-SiR¹₂H, where
R¹ is a monovalent hydrocarbon group and
x ≥ 1, alternatively x may range from 1 to 400,
alternatively x may range from 5 to 100.
R¹ may be a substituted or unsubstituted aliphatic or aromatic hydrocarbon. Monovalent unsubstituted aliphatic hydrocarbon groups are exemplified by, but not limited to alkyl groups such as methyl, ethyl, propyl, pentyl, octyl, undecyl, and octadecyl and cycloalkyl groups such as cyclohexyl. Monovalent substituted aliphatic hydrocarbon groups are exemplified by, but not limited to halogenated alkyl groups such as chloromethyl, 3-chloropropyl, and 3,3,3-trifluoropropyl. The aromatic hydrocarbon group is exemplified by, but not limited to, phenyl, tolyl, xylyl, benzyl, styryl, and 2-phenylethyl.

The linear, SiH terminated, organohydrogenpolysiloxane are exemplified by SiH terminated polydimethylsiloxanes comprising the general formula;

(CH₃)₂HSiO[(CH₃)₂SiO]ₓSiH(CH₃)₂,

where x is defined above.

Component (A) may be an organohydrogensiloxane having at least one, or alternatively at least two SiH containing cyclosiloxane rings in its molecule. Cyclosiloxane rings contain at least three siloxy units (that is the minimum needed in order to form a siloxane ring), and may be any combination of (R₃SiO_{0.5}), (R₂SiO), (RSiO_{1.5}), or (SiO₂) siloxy units that forms a cyclic structure, providing at least one of the cyclic siloxy units on each siloxane ring contains one SiH unit, that is there is at least one (R₂HSiO_{0.5}), (RHSiO), or a (HSiO_{1.5}) siloxy unit present in the ring. These siloxy units can be represented as M^{H}, D^{H}, and T^{H} siloxy units respectively when R is methyl.

In one embodiment, component A) is an organohydrogencyclosiloxane (a) having at least two SiH units on the siloxane ring which may contain any number of siloxy units (as defined above) provided there are at least two SiH units on the cyclosiloxane ring. For example, the cyclic siloxane can comprise any number of M, M^{H}, D, D^{H}, or T^{H} siloxy units. Representative, non-limiting examples of such organohydrogencyclosiloxanes useful to prepare component (A) have the average formula D^{H}ₐD_{b} where a is ≥ 1 and b is ≥ 0, and a + b ≥3. Alternatively, the organohydrogencyclosiloxane may be selected from those having the formula [(CH₃)HSiO]_{g} where g is 3 - 8, such as D^{H}₄ , D^{H}₅, D^{H}₆, or mixtures thereof.

In another embodiment, component A) the organohydrogensiloxane has at least two SiH containing cyclosiloxane rings in its molecule, the cyclosiloxane rings of the organohydrogensiloxane may be linked together by a divalent organic or siloxane group, or combination thereof. The divalent linking group may be designated as Y and the cyclosiloxane as G. Thus, the such organohydrogensiloxane may be represented by the general formula G-[Y-G]ₐ, where G is a cyclosiloxane as described above and Y is a divalent organic, a siloxane, a polyoxyalkylene group, or combination thereof, and the subscript a is greater than zero. Organohydrogensiloxane having at least two SiH containing cyclosiloxane rings may be prepared via a hydrosilylation reaction of
a) an organohydrogencyclosiloxane having at least two SiH units on the siloxane ring and, D) a compound or mixture of compounds having at least two aliphatic unsaturated groups in its molecule. The organohydrogencyclosiloxane (a) having at least two SiH units on the siloxane ring are the same as defined above. Suitable compounds containing at least two aliphatic unsaturated hydrocarbon groups in its molecule are described below as component D).

Hydrosilylation reactions involving organohydrogensiloxanes and unsaturated compounds are well known. Any suitable hydrosilylation catalysts know in the art may be used, or alternatively may be selected from those described below as component C). Any of the known hydrosilylation techniques and reactions may be employed to prepare component A) from a) organohydrogencyclosiloxane having at least two SiH units on the siloxane ring and, D) a compound or mixture of compounds having at least two aliphatic unsaturated groups in its molecule. However, the reaction is conducted in such a manner to provide an organohydrogensiloxane having at least two SiH containing cyclosiloxane rings in its molecule. Typically, the hydrosilylation reaction is conducted with a molar excess of the a) the organohydrogencyclosiloxane having at least two SiH units on the siloxane ring and D) the crosslinking compound containing at least two terminal aliphatic unsaturated groups in its molecule. The molar excess may be expressed as the molar ratio of SiH units to unsaturated group, such ratio may range from 2/1 to 8/1, alternatively from 2/1 to 6/1, or alternatively from 3/1 to 4/1.

Alternatively, the organohydrogensiloxane useful as component A) may be selected from any of the organohydrogensiloxanes taught in WO03/093349, which is herein incorporated by reference for its teaching of suitable organohydrogensiloxanes.

### B) Vegetable Oil

Component B) is a vegetable oil containing at least one unsaturated group. As used herein, "vegetable oil" means any lipid compound derived from any "natural" source such as a plant or vegetable. Thus, the natural oil may be any liquid plant or vegetable lipids extracted from seeds or nuts. Natural oil also encompasses natural butters, which are solid vegetable lipids at ambient temperature. Many vegetable oils contain esters of fatty acids in the form of triglycerides. Triglycerides are glycerin esterified with either saturated or unsaturated fatty acids. Example triglyceride esters of such fatty acids found in vegetable oils include oleic acid (C18:1), linoleic acid (C18:2) and linolenic acid (C18:3).

Representative, non-limiting examples of vegetable oils suitable as component B) in the present invention include; jojoba oil, soybean oil, safflower oil, linseed oil, corn oil, sunflower oil, canola oil, sesame oil, cottonseed oil, palm oil, rapeseed oil, tung oil, fish oil, peanut oil, sweet almond oil, beautyleaf oil, palm oil, grapeseed oil, arara oil, cottonseed oil, apricot oil, castor oil, alfalfa oil, marrow oil, cashew nut oil, oats oil, lupine oil, kenaf oil, calendula oil, euphorbia oil, pumpkin seed oil, coriander oil, mustard seed oil, blackcurrant oil, camelina oil, tung oil tree oil, peanuts oil, opium poppy oil, castor beans oil, pecan nuts oil, brazil nuts oil, oils from brazilian trees, wheat germ oil, candlenut oil, marrow oil, karate butter oil, barley oil, millet oil, blackcurrant seed oil, shea oil (also known as shea butter), maize oil, evening primrose oil, passionflower oil, passionfruit oil, quinoa oil, musk rose oil, macadamia oil, muscat rose oil, hazelnut oil, avocado oil, olive oil or cereal (corn, wheat, barley or rye) germ oil and combinations thereof.

In one embodiment, the jojoba oil is selected as the vegetable oil. Jojoba oil is primarily composed by non-branched monoesters derived from mono-unsaturated alcohols and fatty acids, which represent 97% of the final composition. The most abundant isomers are docos-cis-13-enyl eicos-cis-11-enoate (37%) and eicos-cis-11-enyl octadec-cis-9-enoate (24%), representing over 50% of the esters complex blend. The oil typically does not have more than 2% of free fatty acids.

In another embodiment, the vegetable oil is sesame oil. The sesame plant is similar in type to oil-seed rape and is cultivated in particular in the East Indies. The oil content of the seeds is 45-54%. It is the refined triglyceride oil obtained from the seeds of *Sesamum Indicum.* The fatty acid esters of the triglycerides consist primarily esters of linoleic acid and oleic acid in an approximate ratio of 1:1

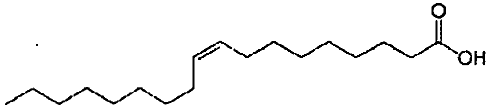

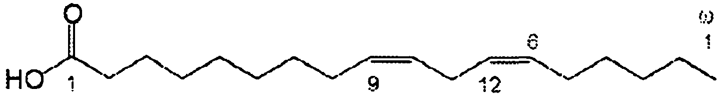

In other specific embodiments, the vegetable oil is selected from shea butter, evening primrose oil, castor oil, almond oil, rice bran oil, sunflower oil, grape seed oil, rapeseed oil, palm oil, or mixtures thereof. Compositional data regarding the type and amounts of internal double bonds are further disclosed in the Examples section below.

### C) Hydrosilylation Catalyst

Ingredient (C) is a hydrosilylation catalyst. Ingredient (C) is added in an amount sufficient to promote reaction between the organohydrogensiloxane and the vegetable oil. However, the amount of ingredient (C) may range from 0.01 to 1,000 ppm, alternatively 0.01 to 100 ppm, and alternatively 0.01 to 50 ppm of platinum group metal based on the weight of the composition.

Suitable hydrosilylation catalysts are known in the art and commercially available. Ingredient (C) may comprise a platinum group metal selected from the group platinum, rhodium, ruthenium, palladium, osmium or iridium metal or organometallic compound thereof, and a combination thereof. Ingredient (C) is exemplified by compounds such as chloroplatinic acid, chloroplatinic acid hexahydrate, platinum dichloride, and complexes of said compounds with low molecular weight organopolysiloxanes or platinum compounds microencapsulated in a matrix or coreshell type structure. Complexes of platinum with low molecular weight organopolysiloxanes include 1,3-diethenyl-1,1,3,3-tetramethyldisiloxane complexes with platinum. These complexes may be microencapsulated in a resin matrix. Alternatively, the catalyst may comprise 1,3-diethenyl-1,1,3,3-tetramethyldisiloxane complex with platinum. When the catalyst is a platinum complex with a low molecular weight organopolysiloxane, the amount of catalyst may range from 0.02 to 0.2 parts by weight based on the total weight of the composition.

Suitable hydrosilylation catalysts for ingredient (C) are described in, for example, U.S. Patents 3,159,601; 3,220,972; 3,296,291; 3,419,593; 3,516,946; 3,814,730; 3,989,668; 4,784,879; 5,036,117; and 5,175,325 and EP 0 347 895 B. Microencapsulated hydrosilylation catalysts and methods of preparing them are also known in the art, as exemplified in U.S. Patent No. 4,766,176; and U.S. Patent No. 5,017,654.

### D) The crosslinking compound containing at least two terminal aliphatic unsaturated groups in its molecule

Component D) is a compound, or any mixture of compounds, containing at least two terminal aliphatic unsaturated groups in its molecule. The compound may be any diene, diyne or ene-yne compound. Diene, diyne or ene-yne compounds are those compounds (including polymeric compounds) wherein there are at least two terminal aliphatic unsaturated groups with some separation between the groups within the molecule. The unsaturation groups are at the termini of the compound, or pendant if part of a polymeric compound. Compounds containing terminal or pendant unsaturated groups can be represented by the formula R²-Y-R² where R² is a monovalent unsaturated aliphatic hydrocarbon group containing 2 to 12 carbon atoms, and Y is a divalent organic or siloxane group or a combination of these. Typically R² is CH₂=CH-, CH₂=CHCH₂-, CH₂=C(CH₃)CH₂- or CH≡C-, and similar substituted unsaturated groups such as H₂C=C(CH₃) -, and HC≡C(CH₃)-.

Component D) is typically used to increase the molecular weight of the silicone-copolymer reaction products of components A), B), and C). Component D) can be used to form silicone terpolymer or crosslinked structures, including silicone elastomeric materials, as discussed in more detail below.

The compound having the formula R²-Y-R²- as component D) may be considered as being a "organic", "hydrocarbon", "organic polymer", "polyether" or "siloxane", or combinations thereof, depending on the selection of Y. Y may be a divalent hydrocarbon, a siloxane, a polyoxyalkylene, a polyalkylene, a polyisoalkylene, a hydrocarbon-silicone copolymer, or mixtures thereof.

In one embodiment, the component D) is selected from an organic compound, herein denoted as D¹), having the formula R²-Y¹- R² where R² is a monovalent unsaturated aliphatic group containing 2 to 12 carbon atoms and Y¹ is a divalent hydrocarbon. The divalent hydrocarbon Y¹ may contain 1 to 30 carbons, either as aliphatic or aromatic structures, and may be branched or un-branched. Alternatively, the linking group Y¹ in D¹ may be an alkylene group containing 1 to 12 carbons. Component D¹) may be selected from α, ω - unsaturated alkenes or alkynes containing 1 to 30 carbons, and mixtures thereof. Component D¹) may be exemplified by, but not limited to 1,4-pentadiene, 1,5-hexadiene; 1,6-heptadiene; 1,7-octadiene, 1,8-nonadiene, 1,9-decadiene, 1,11-dodecadiene, 1,13-tetradecadiene, and 1,19-eicosadiene, 1,3-butadiyne, 1, 5-hexadiyne (dipropargyl), and 1-hexene-5-yne.

In another embodiment, the component D) is selected from a R²-Y²-R² compound where Y² is a siloxane, herein denoted as D²). The Y² siloxane group may be selected from any organopolysiloxane bonded to at least two organic groups having aliphatic unsaturation, designated as R², to form R²-Y²- R² structures. Thus, component D²) can be any organopolysiloxane, and mixtures thereof, comprising at least two siloxane units represented by the average formula R² RₘSiO_{(3-m)/2}
wherein
R is an organic group,
R² is a monovalent unsaturated aliphatic group as defined above, and
m is zero to 2

The R² group may be present on any mono, di, or tri siloxy unit in an organopolysiloxane molecule, for example; (R²RSiO_{0.5} ), (R²RSiO), or (R²SiO_{1.5}); as well as in combination with other siloxy units not containing an R² substituent, such as (R₃SiO_{0.5}), (R₂SiO), (RSiO_{1.5}), or (SiO₂) siloxy units where R is independently any organic group, alternatively a hydrocarbon containing 1 to 30 carbons, alternatively an alkyl group containing 1 to 30 carbons, or alternatively methyl; providing there are at least two R² substituents in the organopolysiloxane.

Representative, non-limiting, examples of such siloxane based R²-Y²-R² structures suitable as component D²) include;

(R₂R²SiO_{0.5})(SiO₂)_{w}(R₂R²SiO_{0.5})

(R₂R²SiO_{0.5})(SiO₂)_{w}(R₂SiO)ₓ(R₂R²SiO_{0.5})

(R₂R²SiO_{0.5})(R₂SiO)ₓ(R₂R²SiO_{0.5})

(R₃SiO_{0.5})(R₂SiO)ₓ(R²RSiO)_{y}(R₃SiO_{0.5})

(R₃SiO_{0.5})(R₂SiOₓ(R²RSiO)_{y}(RSiO_{1.5})_{z}(R₃)SiO_{0.5})

(R₃SiO_{0.5})(R₂SiO)ₓ(R²RSiO)_{y}(SiO₂)_{w}(R₃SiO_{0.5})

where w ≥ 0, x ≥ 0, y ≥ 2, and z is ≥ 0, R is an organic group, and
R² is a monovalent unsaturated aliphatic hydrocarbon group.

D² may be selected from vinyl functional polydimethylsiloxanes (vinyl siloxanes), such as those having the average formula;

CH₂=CH(Me)₂SiO[Me₂SiO]ₓSi(Me)₂CH=CH₂

Me₃SiO[(Me)₂SiO]ₓ[CH₂=CH(Me)SiO]_{y} SiMe₃

wherein Me is methyl,
x ≥ 0, alternatively x is 0 to 200, alternatively x is 5 to 100,
y ≥ 2, alternatively y is 2 to 200, alternatively y is 5 to 100.
D² may also be selected from hexenyl functional polydimethylsiloxanes. Both hexenyl and vinyl functional polydimethylsiloxanes are known, and there are many commercially available.

In another embodiment, component D) is selected from a polyether compound, herein denoted as D³), having the formula R²-Y³- R² compound where R² is as defined above and Y³ is a polyoxyalkylene group having the formula (CₙH₂ₐO)_{b} wherein n is from 2 to 4 inclusive,
b is greater than 2,
alternatively b can range from 2 to 100,
or alternatively b can range from 2 to 50.
The polyoxyalkylene group typically can comprise oxyethylene units (C₂H₄O), oxypropylene units (C₃H₆O), oxybutylene units (C₄H₈O), or mixtures thereof. Thus, the R²-Y³- R² compound may be selected from a polyoxyalkylene group having the formula R²-O-[(C₂H₄O)_{c} (C₃H₆O)_{d} (C₄H₈O)ₑ]-R² where c, d, and e may each independently range from 0 to 100, providing the sum of c + d + e is greater than 2, alternatively the sum of c + d + e ranges from 2 to 100, or alternatively the sum of c + d + e ranges from 2 to 50.

Alternatively, the polyoxyalkylene group comprises only oxypropylene units (C₃H₆O)_{d}. Representative, non-limiting examples of polyoxypropylene containing R²-Y³- R² compounds include;

H₂C=CHCH₂ O[C₃H₆O]_{d}CH₂CH=CH₂

H₂C=CHO[C₃H₆O]_{d}CH=CH₂

H₂C=C(CH₃)CH₂O[C₃H₆O]_{d}CH₂C(CH₃)=CH₂

HC≡CCH₂O[C₃H₆O]_{d}CH₂C≡CH

HC≡CC(CH₃)₂[C₃H₆O]_{d}C(CH₃)₂C≡CH

where d is as defined above.
Representative, non-limiting examples of polyoxybutylene containing R²-Y³- R² compounds include;

H₂C=CHCH₂O[C₄H₅O]ₑCH₂CH=CH₂

H₂C=CHO[C₄H₈O]ₑCH=CH₂

H₂C=C(CH₃)CH₂O[C₄H₈O]ₑCH₂C(CH₃)=CH₂

HC≡CCH₂O[C₄H₈O]ₑCH₂C≡CH

HC≡CC(CH₃)₂O[C₄H₈O]ₑC(CH₃)₂C≡CH

Component D) may also be a mixture of various polyethers, i.e. a mixture of D³ components.

In another embodiment, component D) is selected from a R²-Y⁴ - R² compound, herein denoted as D⁴), where R² is as defined above and Y⁴ is a polyalkylene group, selected from C2 to C6 alkylene units or their isomers. One example is polyisobutylene group which is a polymer containing isobutylene unit. The molecular weight of the polyisobutylene group may vary, but typically ranges from 100 to 10,000 g/mole. Representative, non-limiting examples of R²-Y- R² compounds containing a polyisobutylene group includes those commercially available from BASF under the tradename of OPPONOL BV, such as OPPONOL BV 5K, a diallyl terminated polyisobutylene having an average molecular weight of 5000 g/mole.

In yet another embodiment, component D) is selected from a R²-Y⁵- R² compound, herein denoted as D⁵), where R² is as defined above and Y⁵ is a hydrocarbon-silicone copolymer group. The hydrocarbon-silicone copolymer group may have the formula

-[R¹ᵤ(R₂SiO)ᵥ]_{q} -

where R¹ and R are as defined above;
u and v are independently ≥ 1, alternatively u ranges from 1 to 20,
alternatively v ranges from 2 to 500, or from 2 to 200,
q is >1, alternatively q ranges from 2 to 500, alternatively q ranges from 2 to 100. R²-Y⁵ - R² compounds having a hydrocarbon-silicone copolymer group may be prepared via a hydrosilylation reaction between an α-ω unsaturated hydrocarbon, such as those described above as D¹, and an organohydrogensiloxane. A representative, non-limiting example of such a reaction is shown below.

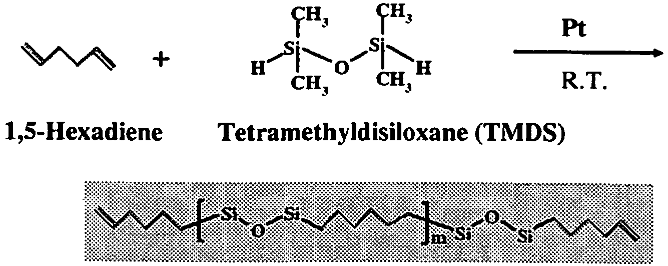

Component D) may also be a mixture of any diene, diyne or ene-yne compound, such as any combinations of D¹, D², D³, D⁴, and D⁵.

The amounts of component a) and component D) used to prepare the present composition will depend on the individual components and the desired SiH to aliphatic unsaturation ratio. The ratio of SiH in component a) to aliphatic unsaturation from component D) useful to prepare the compositions of the present invention can be from 10:1 to 1:10, alternatively 5:1 to 1:5, or alternatively 4:1 to 1:4.

### E) Optional Solvent

The hydrosilylation reaction can be conducted neat or in the presence of a solvent. The solvent can be an alcohol such as methanol, ethanol, isopropanol, butanol, or n-propanol, a ketone such as acetone, methylethyl ketone, or methyl isobutyl ketone; an aromatic hydrocarbon such as benzene, toluene, or xylene; an aliphatic hydrocarbon such as heptane, hexane, or octane; a glycol ether such as propylene glycol methyl ether, dipropylene glycol methyl ether, propylene glycol n-butyl ether, propylene glycol n-propyl ether, or ethylene glycol n-butyl ether, a halogenated hydrocarbon such as dichloromethane, 1,1,1-trichloroethane or methylene chloride, chloroform, dimethyl sulfoxide, dimethyl formamide, acetonitrile, tetrahydrofuran, white spirits, mineral spirits, or naphtha. Organic solvents may be an aliphatic hydrocarbons such as isododecane, isohexadecane, Isopar L (C11-C13 ), Isopar H(C11-C12 ), hydrogentated polydecen. Ethers and esters including isodecyl neopentanoate, neopentylglycol heptanoate, glycol distearate, dicaprylyl carbonate, diethylhexyl carbonate, propylene glycol n butyl ether, ethyl-3 ethoxypropionate, propylene glycol methyl ether acetate, tridecyl neopentanoate, propylene glycol methylether acetate (PGMEA), propylene glycol methylether (PGME). octyldodecyl neopentanoate, diisobutyl adipate, diisopropyl adipate, propylene glycol dicaprylate / dicaprate, and octyl palmitate.

The solvent may also be selected from a volatile methyl siloxane, a volatile ethyl siloxane or a volatile methyl ethyl siloxane having a viscosity at 25°C in the range of 1 to 1,000 mm²/sec such as hexamethylcyclotrisiloxane, octamethylcyclotetrasiloxane, decamethylcyclopentasiloxane, dodecamethylcyclohexasiloxane, octamethyltrisiloxane, decamethyltetrasiloxane, dodecamethylpentasiloxane, tetradecamethylhexasiloxane, hexadeamethylheptasiloxane, heptamethyl-3-{((trimethylsilyl)oxy)}trisiloxane, hexamethyl-3,3,bis{(trimethylsilyl)oxy}trisiloxane pentamethyl{(trimethylsilyl)oxy}cyclotrisiloxane as well as polydimethylsiloxanes, polyethylsiloxanes, polymethylethylsiloxanes, polymethylphenylsiloxanes, polydiphenylsiloxanes.

The amount of solvent can be up to 50 weight percent, but is typically from 20 to 50 weight percent, said weight percent being based on the total weight of components in the hydrosilylation reaction. The solvent used during the hydrosilylation reaction can be subsequently removed from the resulting reaction product mixture by various known methods, or alternatively remain in the composition.

### F) The Alpha Olefin

Component F) is an alpha olefin. The alpha olefin may be alpha olefinic organic or silicone compound. Often, small amounts of SiH will remain in the silicone copolymer or silicone elastomer after the hydrosilylation reaction(s). The resulting silicone copolymer or elastomer can be further reacted with an alpha olefin in a further hydrosilylation reaction to consume any remaining SiH. Typically, the SiH content of the product composition resulting from reaction of components A), B), C) and optionally D) is first measured, and sufficient alpha olefin F) and catalyst C) is added to further react the SiH. Typically the amount of alpha olefin used is a molar excess of the SiH content of the product composition. In one embodiment the alpha olefin is sufficiently volatile enough such that any excess or unreacted olefin may be removed from the resulting product composition such under reduced pressure. In such instances, the alpha olefin may be a low molecular weight alkene such as ethylene, propylene, butylene, and the like. In another embodiment, the alpha olefin is a higher molecular weight alkene, like 1-octene, 1-decene, 1-dodecene, 1-octadecene, or the like. In yet another embodiment, the alpha olefin may contain an organofunctional group such as an alcohol, diol, or epoxy.

Other alpha olefinic organic compounds may be used to react with small un-reacted quantities of SiH in the copolymers. Examples of these compounds, although not limiting to, mono-allyl poly(oxyalkylene), vinyl- or hexenyl-terminated silicones.

### Silicone Copolvmers

The silicone copolymers of the present invention are obtainable as hydrosilylation reaction products of components A), B), C), optionally D) and optionally F), each as defined above. Any hydrosilylation process may be used to produce the silicone copolymers, and more details are discussed below for typical conditions and certain variations.

Although not wishing to be bound by any theory, the present inventors believe a portion of the reaction products in the hydrosilylation reaction between the organohydrogenpolysiloxane and vegetable oil result from the addition of the SiH units of the organohydrogenpolysiloxane across the internal carbon - carbon double bonds of the various fatty esters present in natural vegetable oils. These fatty esters may be triglycerides based compounds, as reflected by the compositions of the selected vegetable oil, or mono fatty esters, such as those found in jojoba oil. Thus the reaction product contains a least one non-hydrolyzable Si-C bond which links the vegetable oil based fatty ester, designated herein as E, with the organopolysiloxane. Thus, the reaction products of the silicone copolymers contain at least one siloxy unit of the average formula R¹ₘ (E)SiO_{(3-m)/2} where;
R¹ is a monovalent hydrocarbon group,
E is a fatty ester group attached to silicon in the structural unit by at least one
Si-C bond wherein the carbon atom of the bond is one of the carbon
atoms originally present in the aliphatic unsaturated bond of the vegetable oil, m may vary from 0 to 2.
As used herein, "fatty ester" denotes any ester functional organic group found in naturally occurring vegetable oil compounds, typically as either a triglyceride or monoglyceride. The triglycerides or monoglycerides are the glycerin based esters of fatty acids. Such fatty ester vegetable oil compounds generally contain 16 to 50 carbon atoms each with at least one internal double bond per molecule.

Representative, non-limiting examples of the fatty ester group E include; palmitate, margar, stearate, oleate, linoleate, linolenate, gadoleate, eurcate, and tetracosenoate.

In another embodiment, the silicone copolymer comprises an organopolysiloxane containing at least one structural unit of the average formula

R¹ₘSiO_{(3-m)/2} (E) SiO_{(3-m)/2} R¹ₓ

where;
R¹ is a monovalent hydrocarbon group,
E' is a fatty ester group attached to silicon in the structural unit by at least one
Si - C bond wherein the carbon atom of the bond is one of the carbon
atoms originally present in the aliphatic unsaturated bond of the vegetable oil, m may vary from 0 to 2.

Representative, non-limiting examples of the fatty ester group E' include those containing at least two internal double bonds such as linoleate or linolenate.

In a further embodiment, the organopolysiloxanes containing at least one structural unit having the formula R¹ₘ (E)SiO_{(3-m)/2} or R¹ₘSiO_{(3-m)/2} (E) SiO_{(3-m)/2} R¹ₓ may further contain SiH functional siloxy units. Thus the organopolysiloxane reaction products may also be considered an organohydrogensiloxane.

In one embodiment, the reaction product comprises a structural unit of the average formula

E-R¹₂SiO-[R¹₂SiO]ₓ-SiR¹₂R²

where E is a fatty ester as defined above,
R¹ is a monovalent hydrocarbon group as defined above
R² is hydrogen or E.

The hydrosilylation reaction between the vegetable oil and organohydrogenpolysiloxane can occur by the addition of two unsaturations present in either the same ester molecule or different ester molecules, regardless the number of carbon atoms, with the possible formation of block copolymers (ABA), where A would be the previously unsaturated ester. A representative, non limiting example of such a reaction product is; 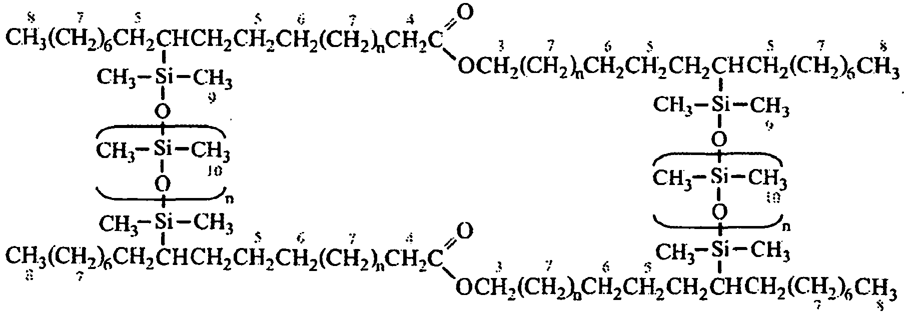 Furthermore, the formation of a network structure where each double bond carbon-carbon of the same oil molecule reacts with a Si-H bond of a different silicone molecule is possible.

The following structures provide representative, non-limiting examples of the organopolysiloxanes and organohydrogensiloxanes structures that may result as silicone copolymer reaction products;

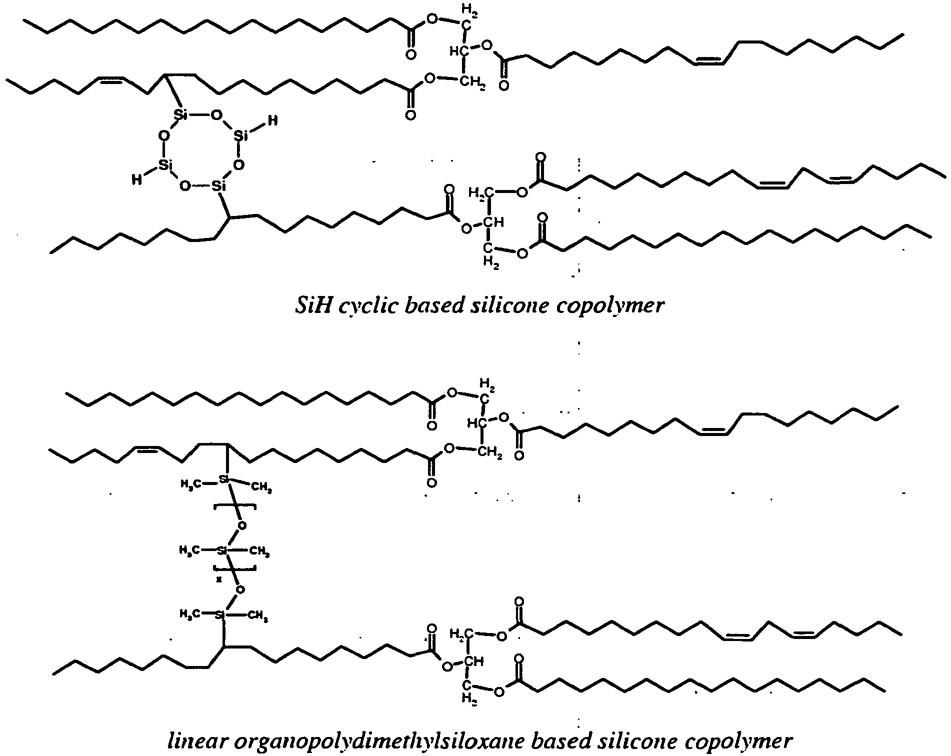

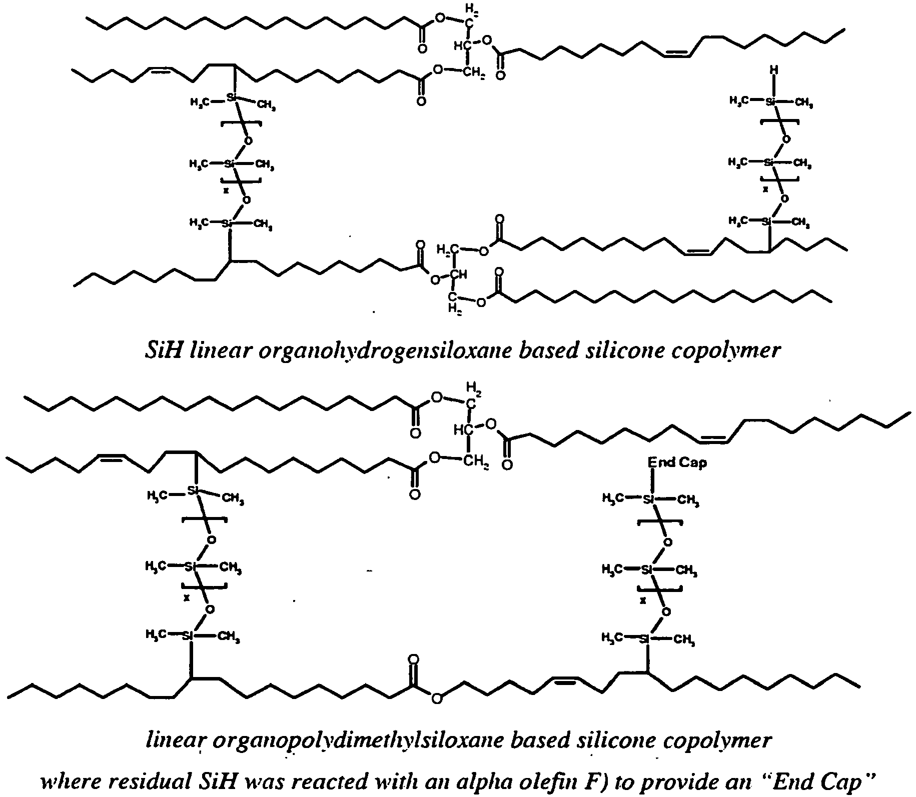

The compositions containing the reaction products of A), B), C), and optionally D) and F) may contain at least 5 weight percent, alternatively 10 wt %, or alternatively 30 wt % of the silicone copolymer in the composition.

### Process for preparing Silicone Copolymers and Elastomers

The silicone copolymers and elastomers of the present invention are obtainable as hydrosilylation reaction products of components A), B), C), and optionally D) and F) as defined above.

The hydrosilylation reaction may be conducted in the presence of a solvent, and the solvent subsequently removed by known techniques. Alternatively, the hydrosilylation may be conducted in a solvent, where the solvent remains in the resulting compositions as a carrier fluid.

The amount of components A), B), and optionally D) used in the hydrosilylation reactions to prepare the silicone copolymer and elastomers can vary, and typically the amounts used are expressed as the molar ratio of the unsaturated group in the reaction mixture from component B) (and additionally component D) when present) vs the SiH content of component A). Typically, the hydrosilylation reaction is conducted with a slight molar excess of the unsaturated groups vs SiH to ensure complete consumption of the SiH in the hydrosilylation reaction. Typically, the hydrosilylation reaction is conducted with a 20%, alternatively 10%, alternatively 5%, or alternatively 1% molar excess of the unsaturated group vs the molar SiH content of the organohydrogenpolysiloxane.

The temperature of the hydrosilylation reaction may vary, but typically the reaction is conducted at elevated temperatures ranging from 80°C to 150°C. Typically, component B) is reacted with component A) at a temperature ranging from 100°C to 150°C.

The hydrosilylation reaction may occur in a single step reaction, or as a series of reactions. For example, components A) and B) may be first reacted in such a manner to form a silicone copolymer containing SiH units (such as when there is a molar excess of SiH in the reaction mixture or when some of the more stable C=C bonds in oils do not react). The resulting SiH containing silicone copolymer may then be further reacted with component D) or F), as further described herein. Alternatively, components A) and D) may first be reacted to form a partially crosslinked organohydrogensiloxane (with a molar excess of SiH of component A) to the unsaturated groups of component D), which is subsequently reacted with component B) to form a silicone copolymer or elastomer.

In one embodiment of the process to prepare the silicone modified vegetable oils, the hydrosilylation reaction between components A) and B) is performed in the absence of organic compounds or polymers having terminal aliphatic unsaturated groups. As discussed above, the vegetable oils used in the process contain internal unsaturated carbon - carbon double bonds. Generally, the rates for hydrosilylation of terminal carbon - carbon double bonds is much faster than internal carbon - carbon double bonds. Thus, it is preferred to conduct the hydrosilylation reaction of components A) and B) in the absence of any hydrocarbons containing terminally unsaturated carbon double bonds.

Alternatively, the silicone copolymers, and in particular silicone elastomers, may be prepared by a process comprising:
I) reacting;
   a) an organohydrogencyclosiloxane having at least two SiH units on a siloxane ring, B) a vegetable oil containing at least one aliphatic unsaturated bond,
   C) a hydrosilylation catalyst,
      in the presence of
   E) an optional solvent,
      wherein the reaction product still contain some un-reacted SiH units,
II) further reacting the product of step I) with;
   b) a compound containing at least two aliphatic unsaturated groups in its molecules,
   C) the hydrosilylation catalyst,
      in the presence of
   E) an optional solvent,
to form the silicone copolymer or elastomer.

Components a, A), B), C) are the same as those described above. Also, the reaction may be conducted under similar conditions as described above.
In aforementioned step II) the molar ratio of the SiH units of component A) to the aliphatic unsaturated groups of component B) ranges from 10/1 to 1/10, alternatively from 5/1 to 1/5,
or alternatively from 4/1 to 1/4.

### Gelled compositions containing the Silicone Elastomer

The silicone elastomers can be added to a carrier fluid (such as the solvents described above as component E) to form gelled compositions, or alternatively be prepared first in a separate reaction and then added to the carrier fluid to obtain a gel. The gelled compositions of the present invention may be characterized by their hardness or firmness. Useful tests to characterize the gels are those recommended by the *Gelatin Manufacturers Institute of America* such as the use of a "Texture Analyzer" (model TA.XT2, Stable Micro Systems, Inc., Godalming, England). Further details and description of the test methods used to characterize the gels can be found in WO 2007/109240, WO 2007/109260, and WO 2007109282.

The silicone gels of the present invention has a compression hardness of at least 200 Newton / m², alternatively 400 Newton / m²_, or alternatively 600 Newton / m².

### Gel Paste compositions containing the Silicone Elastomer

The gelled compositions of the present invention can be used to prepare gel paste compositions containing actives by;
I) shearing the silicone elastomer, as described above,
II) combining the sheared silicone elastomer with additional quantities of
   E) the solvent, as described above, and
   G) optionally, a personal or health care active active
to form a gel paste composition.

The silicone polyether elastomer gel compositions of the present invention blends may be considered as discrete crosslinked silicone elastomer gel particles dispersed in carrier fluids. Thus, the silicone elastomer compositions are effective rheological thickeners for lower molecular weight silicone fluids. As such they can be used to prepare useful gel blend compositions, such as "paste" compositions.

To make such silicone elastomer blends, the aforementioned silicone elastomer gels of known initial elastomer content (IEC) are sheared to obtain small particle size and further diluted to a final elastomer content (FEC). "Shearing", as used herein refers to any shear mixing process, such as obtained from homogenizing, sonalating, or any other mixing processes known in the art as shear mixing. The shear mixing of the silicone elastomer gel composition results in a composition having reduced particle size. The subsequent composition having reduced particle size is then further combined with a carrier fluid. The carrier fluid may be any solvent as described above, but typically is a volatile methyl siloxane, such as D5. The technique for combining the carrier fluid with the silicone elastomer composition having reduced particle size is not critical, and typically involves simple stirring or mixing. The resulting compositions may be considered as a paste, having a viscosity greater than 100,000 cP (mPa·s).

The silicone gels and gel pastes may be combined with various personal or health care actives. Representative, non-limiting examples of such personal or health care actives are disclosed in WO 2007/109240, WO 2007/109260, and WO 2007109282, which are incorporated by reference for their teaching of suitable personal or health care actives.

The silicone modified vegetable oils may be incorporated into personal care formulations such as; an antiperspirant, deodorant, skin cream, skin care lotion, moisturizer, facial treatment, wrinkle remover, facial cleansers, bath oils, sunscreens, pre-shave, after-shave lotions, liquid soap, shaving soap, shaving lather, hair shampoo, hair conditioner, hair spray, mousse, permanent, hair cuticle coat, make-up, color cosmetic, foundation, blush, lipstick, lip balm, eyeliner, mascara, nail polishes, and powders.

The personal care compositions of this invention may be in the form of a cream, a gel, a powder, a paste, or a freely pourable liquid. Generally, such compositions can generally be prepared at room temperature if no solid materials at room temperature are presents in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The compositions according to this invention can be used by the standard methods, such as applying them to the human body, e.g. skin or hair, using applicators, brushes, applying by hand, pouring them and/or possibly rubbing or massaging the composition onto or into the body. Removal methods, for example for color cosmetics are also well known standard methods, including washing, wiping, peeling and the like. For use on the skin, the compositions according to the present invention may be used in a conventional manner for example for conditioning the skin. An effective amount of the composition for the purpose is applied to the skin. Such effective amounts generally range from 1mg/cm² to 3 mg/cm². Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the compositions according to the invention on hair may use a conventional manner for conditioning hair. An effective amount of the composition for conditioning hair is applied to the hair. Such effective amounts generally range from 1 g to 50 g, typically from 1 g to 20 g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit.

### EXAMPLES

These examples are intended to illustrate the invention to one of ordinary skill in the art and should not be interpreted as limiting the scope of the invention set forth in the claims. All measurements and experiments were conducted at 23°C, unless indicated otherwise.

### Materials

### Vegetable oils

Jojoba oil, sesame oil, primrose oil, shea butter, castor oil, rapeseed oil, palm oil, were used as supplied by Lipo Chemicals (207 Nineteenth Avenue, Paterson, NJ 07504). The vegetable oil blend describe in the following examples was a blend of 75 wt% rapeseed oil and 25 wt% palm oil. Table 1 and 2 below summarize the composition of the oils used in these representative examples.

**Table 1. Selected natural oil composition**

| **Product name** | **Shea Butter** | **Jojoba Oil** | **Vegetable Oil Blend** | **Evening Primrose Oil** |
|---|---|---|---|---|
| Specific gravity | 0.91 | 0.86 | 0.92 | 0.92 |
| Comments | Melting Point 30C | | 75% rapeseed oil, 25% palm oil | |
| Palmitic (C16:0) | 6 | 2 | 15 | 6 |
| Margar (C17:0) | | | | |
| Stearic (C18:0) | 40 | | 2 | 2 |
| Arachidic (C20:0) | | | | |
| Behenic (C22:0) | | | | |
| **Oleic (C18:1)** | **44** | **12** | **55** | **8** |
| **Linoleic (C18:2)** | **6** | **3** | **13** | **73** |
| **Linolenic (C18:3)** | | **2** | | **11** |
| **Gadoleic (C20:1)** | | **70** | | |
| **Erucic (C22:1)** | | **15** | | |
| **Tetracosenoic (C24:1)** | | **2** | | |
| **Total % accounted** | 96.0 | **106.0** | 96.0 | 100.0 |
| AOCS CA5A-40 Free fatty acids | 0-1 | 0-1 | 0-1 | 0-1.5 |
| AOCS C8-53 Peroxide value | 0-5 | 0-7 | 0-5 | 0-5 |
| **Iodine value (ASTM D1959097)** | **50-70** | **73-88** | **90-115** | **150-170** |
| Averaged m.w. | 281.55 | 309.89 | 263.20 | 279.02 |
| **Averaged no. of unsaturated bonds** | **0.583** | **1.047** | **1.03** | **1.87** |
| **Wt.% unsaturated C=C bond** | **5.39** | **8.79** | **10.19** | **17.43** |

**Table 2. Typical composition and % unsaturation of selected oils and butter**

| **Natural oil name** | **Castor Oil** | **Almond Oil, Sweet** | **Rice Bran Oil** | **Sunflower Oil** | **Grape seed Oil** |
|---|---|---|---|---|---|
| **Typical composition in weight part** | | | | | |
| **Palmitic (C16:0)** | 1 to 2 | 4-9 | 16-25 | 5-8 | 6-9 |
| **Margar (C17:0)** | < 0.1 | < 0.2 | | | |
| **Stearic (C18:0)** | < 1.5 | 0.5-3 | 0.5-3.5 | 2.5-7 | 3-6 |
| **Arachidic (C20:0)** | <0.6 | <0.2 | <0.5-2 2 | <0.5 | <0.5 |
| **Behenic (C22:0)** | | | <0.8 | 0.1-3 | <0.3 |
| **Palmitoleic acid (C16:1)** | <0.5 | <0.6 | <1.0 | <0.5 | <1 |
| **Oleic (C18:1)** | **3 to 7 and 80 -91 of C18:1 OH** | 62-86 | 35-50 | 15-40 | **12-25** |
| **Gadoleic (C20:1)** | | <0.3 | **<1.0** | <0.5 | |
| **Erucic (C22:1)** | | <0.1 | | | |
| **Linoleic (C18:2)** | 5 to 7 | 20-30 | 25-40 | 40-74 | 60-75 |
| **Linolenic (C18:3)** | | <0.4 | 0.5-3 | <0.3 | < 1.5 |

| **Composition summary** | | | | | |
|---|---|---|---|---|---|
| **Total accounted for; weight parts** | 99.30 | 108.20 | 101.90 | 93.20 | 99.65 |
| **Mono-unsaturated total** | 90.8 | 74.2 | **43.0** | **23.0** | 19.0 |
| **Poly-unsaturated total** | . 6.0 | 25.2 | 33.8 | 57.1 | 68.5 |
| **Unsaturated total = mono-unsaturated + 2 x polyunsaturated)** | 102.8 | 124.4 | 110.6 | 137.2 | 156.0 |
| **Iodine value (AOCS CD 1-25 method)** | 85-95 | 95-109 | 88-105 | 120-145 | 130-145 |
| **Average m.w., g/mole** | 281.98 | **280.48** | 277.15 | 280.55 | 279.22 |
| **Averaged no. of unsaturated bonds per molecule** | 1.035 | **1.153** | 1.111 | 1.475 | 1.569 |
| **Wt.% unsaturated** | 9.54 | 10.69 | 10.43 | 13.67 | 14.6 |

The composition information shown in Table 1 and 2 "composition summary" are tabulated or derived based on the typical composition shown in the table. The C=C unsaturation content of the oils are expressed in terms of "mono-unsaturated total," "polyunsaturated total," and "unsaturated total."

The average number of unsaturated C=C bonds per molecule and the wt. % unsaturated C=C bond are also calculated. These are the quantitative properties that are useful to express the extent of unsaturation in oil. The "average number of unsaturated bonds" was calculated based on the information found in open literature to a given ester segment, without considering whether it is a monoester or a part of triglyceride molecule. This also applies to the "wt. % unsaturated C=C bond definition. The iodine value also indicates the extent of unsaturation in a given natural oil.

### Organohydrogensiloxanes

SiH SILOXANE 1 - a dimethyl, hydrogen-siloxy terminated polydimethylsiloxane having the average formula of M^{H}D₁₇M^{H}.
SiH SILOXANE 2 A dimethyl, hydrogen-siloxy terminated polydimethylsiloxane having the average formula of M^{H}D₆M^{H}.
SiH SILOXANE 3 - an organohydrogensiloxane having at least two SiH containing cyclic rings, prepared according to the process described in Example 1C of WO 2007/109240. MeH CYCLICS = methylhydrogen cyclosiloxanes (MeH cyclics) having the formula [(CH₃)HSiO)ₓ where the average value of x is 4.4.

### Other components

SYL-OFF® 4000 CATALYST (Dow Coming Corporation, Midland, MI) was used as the hydrosilylation catalyst, which is an organo-platinum vinyldimethicone compound.
VINYL SILOXANE = a dimethylhexenylsiloxy-terminated dimethylpolysiloxane of the general formula (CH₂=CH(CH₂)₄)(CH₃)₂SiO[(CH₃)₂SiO]_{dp}Si(CH₃)₂((CH₂)₄(CH₂=CH)), where the average degree of polymerization (dp) was 37 and a viscosity of 40 mm²/s at 25°C.
VINYL SILOXANE # 2= a dimethylvinylsiloxy-terminated dimethylpolysiloxane of the general formula (CH₂=CH)(CH₃)₂SiO[(CH₃)₂SiO]_{dp}Si(CH₃)(CH=CH₂), where the average degree of polymerization (dp) was 8 and having a viscosity of 4 mm²/s at 25°C.
Si DIENE = a silicone hydrocarbon copolymer with diallyl functionalities at ends (*α*, ω-dihexenyl hydrocarbon oligomers) prepared by reacting 1,5-hexadiene and tetramethylsiloxane (TMDS) according to the procedures described in WO 2007/109282 (Example 2).
PO20 - Polycerin DUS-80 = α,ω -diallyl polypropylene oxide having 20 propylene oxide (PO) units from NOF Corporation (Japan).
D5 CYCLICS - decamethylcyclopentasiloxane (DC245 Fluid, supplied by Dow Coming Corporation).
IDD = ISODODECANE (Permethyl 99A from Presperse Incorporated, Somerset, New Jersey)
ALPHA OLEFIN 1: 1-Octene, Cheveron Phillips Chemical Compangy (The Woodlands, Texas)

### Example 1

### Silicone modified jojoba oil

A 150 mL beaker was charged with 17.9g of D5 CYCLICS (48.35 mmol), 15g of SiH SILOXANE 1 organohydrogenpolysiloxane (9.74 mmol) and 6.30 g of JOJOBA OIL (10.40 mmol approximately). In a separate container, 0.019g of Syl-Off® 4000 Catalyst (10 ppm of active platinum) was blended with 3.50g of D5 CYCLICS (9.45 mmol). The blend containing jojoba oil was heated to 95-100°C, using a silicone bath, under constant agitation at 250 rpm, within an open container. After homogenization for 10 minutes, the Syl-Off® 4000 catalyst blend was added to the initial system, and both agitation and temperature were maintained for at least 5 continuous hours. After this period, the mix was cooled under slow agitation up to a white viscous fluid was formed. IR and NMR analysis of the reaction product showed successful addition of the SiH across the C=C bonds present in the starting jojoba oil.

### Example 2

### Silicone modified sesame oil

A reaction flask was charged with 30.0g of D5 CYCLICS, 15 g of the SiH SILOXANE 1 organohydrogenpolysiloxane (9.74 mmol) and 10.27 g sesame oil (10.40 mmol approximately). In a separate container, 0.019g of Syl-Off® 4000 Catalyst (10 ppm of active platinum) was blended with 3.50g of D5 CYCLICS (9.45 mmol). The blend containing sesame oil was heated to 95-100°C, using a silicone bath, under constant agitation at 250 rpm, within an open container. After homogenization for 10 minutes, the Syl-Off® 4000 catalyst blend was added to the initial system, and both agitation and temperature were maintained for at least 5 continuous hours at 95 to 105°C. IR and NMR analysis of the reaction product confirmed successful addition of the SiH across the C=C bonds present in the starting sesame oil.

### Example 3

### Silicone modified jojoba oil as a hair care additive

The following "base" hair conditioner formulation was used in these examples.

| **Ingredient** | **%t.** |
|---|---|
| Water | 93.24 |
| Hydroxyethylcelulose | 1.50 |
| Cetrimonium Chloride | 2.00 |
| Cetearyl Alcohol | 3.00 |
| Phenoxyethanol + Parabens | 0.20 |
| Citric Acid | 0.06 |

Jojoba oil, or silicone modified jojoba oil (as prepared in Example 1 and denoted in this example as SilNat oil), were evaluated in the base hair conditioner formulation according to the following compositions.

| **Ingredient** | **% wt.** | | |
|---|---|---|---|
| | **A** | **B** | **C** |
| Base | 96.00 | 96.00 | 97.40 |
| | | | |
| Jojoba Oil | | 2.00 | 0.60 |
| DC-245 Fluid | | 2.00 | 2.00 |
| | | | |
| SilNat Jojoba | 4.00 | | |

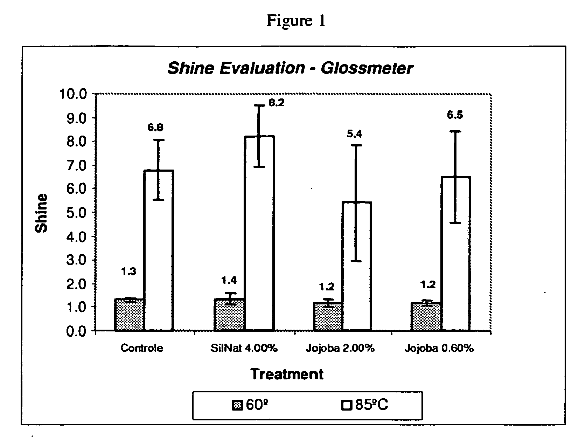

The hair conditioner formulations were applied in caucasian hair tresses supplied by DeMeo Brothers, with dimensions 2.5cm width and 25cm length. After been treated, these tresses were evaluated for shine properties using a glossmeter, at 60° and 85°. The results are summarized in figure 1, showing that the silicone modified jojoba oil having improved gloss vs the control or unmodified jojoba oil.

The same Caucasian hair tresses were also evaluated by a panel (comprised of ten testers), for various sensory attributes, using ranking evaluation. Each one of the testers was asked to rank each tress and attribute in a range of 1 to 5. The panel found:
a) SilNat increased wave intensity of hair tresses at 95% confidence level, when compared to control and to pure jojoba oil.
b) SilNat facilitates detangling when compared to control and to pure jojoba oil, at 95% confidence level.
c) SilNat also facilitates combing, confirming what was found in the instrumental analysis (95% confidence interval).
d) Improves shine when compared to control, at 90% confidence level. Provides more shine than formulation with 2.00% pure oil
   (95% confidence interval).
e) Tendency to increase body / fullness of hair tresses, compared to non-modified jojoba oil.

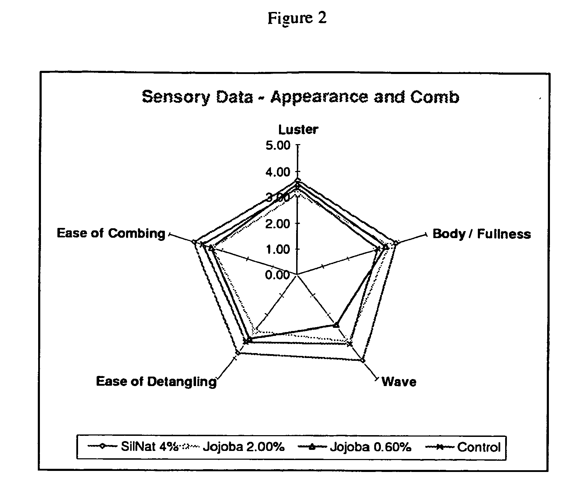

### Results are expressed in the figure 2 (in terms of actives).

The silicone modified jojoba oil was evaluated in a leave on conditioning formulation. Solutions made with DC-245 Fluid were evaluated and concentrations were doubled, to test different conditions
- SilNat = 4.00% actives
- Jojoba Oil = 4.00% and 1.20% actives

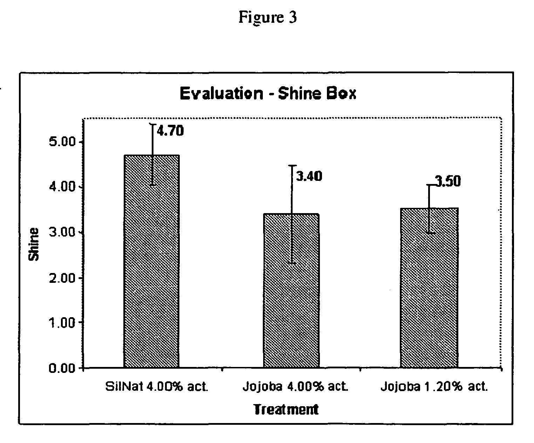

Hair tresses were prepared according to global protocols, using Caucasian hair tresses supplied by DeMeo Brothers, with dimensions 2.5cm width and 25cm length. Treated hair tresses were submitted to a panel of 10 persons, using a shine box device (reference US patent 5,419,627) and ranking methodology. Each panelist was asked to rank hair tresses in terms of shine intensity, in a range of 1 (less shiny) to 5 (more shiny). SilNat results were compared to pure non-modified jojoba oil. The results, as shown in figure 3, indicate SilNat increased shine when compared to pure jojoba oil, at both concentrations at the 95% confidence level.

### Example 4

### Silicone-vegetable oil copolymers containing SiH

Two silicone-vegetable oil copolymers were prepared from MeH CYCLICS and vegetable oils via Pt-catalyzed hydrosilylation reaction in IDD solvent as summarized in the table below. The reaction was carried out at 120°C for 5 hours. The [SiH] of the starting mixture and the reacted mixture were quantified using FTIR. The actual % of SiH indicates extent of reaction with unsaturated double bonds in natural oils.

| **Silicone-vegetable oil copolymers containing SiH** | | |
|---|---|---|
| **Example #** | **4A** | **4B** |
| **Batch description** | **MeH CYCLICS +** V**egetable oil @ 0.60 SiH/Vi in IDD; 87.6% Vegetable oil in solids** | **MeH CYCLICS + Vegetable oil @ 0.80 SiH/Vi in IDD; 84% Vegetable oil in solids** |
| **A)organohydrogensiloxane** | MeH CYCLICS | MeH CYCLICS |
| **B) Natural oil** | **Vegetable oil** | **Vegetable oil** |
| **Wt.% natural oil in copolymer** | **87.6** | 84.1 |
| **Carrier fluid type** | **IDD** | **IDD** |
| **SiH:Vi ratio** | 0.60 | 0.80 |
| **% Polymer conc.** | 70.0 | 70.0 |

| **Actual amount** | | |
|---|---|---|
| MeH CYCLICS, g | 26.02 | 22.22 |
| **Vegetable oil, g** | 185.99 | 117.80 |
| Isododecane, g | 90.09 | 60.08 |
| Syl-Off 4000, g | 0.30 | 0.25 |
| Total Batch, g | 302.40 | 200.35 |
| **Appearance of starting mixture** | **Clear yellow liquid** | **Clear light yellow liquid** |
| **Appearance of reacted mixture after 5 hrs** @ 120C | **Yellow, slightly hazy liquid, moderate viscosity** | **Light yellow, slightly hazy liquid** |
| **Wt.**% **SiH in the unreacted mixture, actual** | **0.1438** | **0.1954** |
| **Wt.% SiH in the final mixture (after 5 hrs** @ **120C)** | **0.0489** | **0.0889** |
| % **of SiH reacted / consumed** | **65.99%** | **54.50%** |

### Example 5

### Silicone-shea buffer copolymers containing SiH

Two silicone-shea butter copolymers prepared from MeH CYCLICS and shea butter via Pt-catalyzed hydrosilylation reaction in IDD solvent as summarized in the table below. The reaction was carried out at 120°C for 5 hours. The [SiH] of the starting mixture and the reacted mixture were quantified using FTIR. The actual % of SiH consumed is considered an indicative of the extent of reaction with unsaturated double bonds in natural oils.

| Silicone-shea butter copolymers containing SiH | | |
|---|---|---|
| **Example** # | **5A** | **5B** |
| **Batch description** | MeH CYCLICS + **Shea Butter** @ **0.80 SiH/; 91 % Shea Butter in solids; 50% solids in IDD** | MeH CYCLICS + **Shea Butter** @ **0.60 SiH/Vi; 93% Vegetable oil in solids; 50% solids in IDD** |
| **A)organohydrogensiloxane** | MeH CYCLICS | MeH CYCLICS |
| **B) Natural oil** | **Shea Butter** | **Shea Butter** |
| **Wt**.% **natural oil in copolymer** | 90.9 | 93.0 |
| **Carrier fluid type** | **IDD** | **IDD** |
| **SiH**:**Vi ratio** | 0.80 | 0.60 |
| % **Polymer conc.** | **50.0** | |

| **Actual amount** | | **50.0** |
|---|---|---|
| MeH CYCLICS, g | 13.62 | 10.44 |
| **Shea Butter, g** | 136.43 | 139.56 |
| Isododecane, g | 150.05 | 150.08 |
| Syl-Off 4000, g | 0.30 | 0.30 |
| Total Batch, g | 300.40 | 300.38 |
| **Appearance of starting mixture** | **Bright yellowish, clear solution** | **Bright yellow, slightly hazy solution** |
| **Appearance of reacted mixture after 5 hrs** @ **120C** | **Much lighter yellowish, clear solution when warm; cooled down to white slushy solids** | **Much lighter yellowish, clear solution when warm; cooled down to white slushy solids** |
| Wt.*%* **SiH in the unreacted mixture, actual** | **0.0748** | **0.0564** |
| Wt. % **SiH in the final mixture (after 5 hrs** @ **120C)** | **0.0479** | **0.0277** |
| % **of SiH reacted / consumed** | **35.96%** | **51.99%** |

### Example 6

### silicone-natural oil copolymers containing SiH

Illustrated in the following tables are two silicone-natural oil copolymers prepared from MeH CYCLICS, VINYL SILOXANE , and shea butter via Pt-catalyzed hydrosilylation reaction in IDD solvent. The reaction was carried out at 120°C for 5 hours. The [SiH] of the starting mixture and the reacted mixture were quantified using FTIR. The actual % of SiH consumed is considered an indicative of the extent of reaction with unsaturated double bonds in natural oils.

| | | |
|---|---|---|
| **Silicone-natural oil copolymers containing SiH** | | |

| **Example #** | **6A** | **6B** |
|---|---|---|
| Neat polymer structure | MeH CYCLICS -/Veg oil / Vinyl siloxane @ 18.5/78.4/3.2 | MeH CYCLICS /Shea Butter /Vinyl siloxane @ 0.8/87.1/2.0 |
| A)organohydrogensiloxane | MeH CYCLICS | MeH CYCLICS |
| **B) Natural oil** | **Vegetable oil** | **Shea Butter** |
| **Wt.% Organics in final polymer** | 78.4 | 87.1 |
| **Carrier fluid type** | **IDD** | **IDD** |
| SiH:Vi ratio: step I/II | 1.0/4.0 | 1.0/4.0 |
| **% Polymer conc.** | **50.0** | **50.0** |

| **Formulation: Step I** | | |
|---|---|---|
| MeH CYCLICS, g | 23.84 | 13.85 |
| **Vegetable oil, g** | 101.16 | |
| **Shea Butter, g** | | 111.15 |
| Isododecane, g | 125.00 | 125.00 |
| Syl-Off 4000, g (20 drops) | 0.20 | 0.20 |
| Total Batch, g | 250.20 | 250.20 |
| **condition for step I:** | 3 hrs @ 120 C | 3 hrs @ 120 C |
| **Wt.**% **SiH in the un-reacted mixture, actual** | 0.1667 | 0.0965 |
| **Wt. % SiH in the step I mixture (after 3 hrs** @ **120C)** | 0.109 | 0.0687 |

| **Formation:Step II** | | |
|---|---|---|
| VINYL SILOXANE (1.95% Vi) | 4.09 | 2.58 |
| Isododecane,g | 4.13 | 2.61 |
| Total Batch, g | 258.42 | 255.39 |
| condition for step II: | 2 hrs @ 80C | 3 hrs @ 80C |
| **Wt**% **SiH in final mixture** | 0.0957 | 0.0632 |
| % of Sih reacted after step I rxn | 34.6% | 28.8% |
| % of total SiH reacted after Step II, | 42.59% | 34.51% |

### Examples 7

### Silicone-Natural oil copolymer derived from linear silicone and sunflower oil

In this example, prescribed amounts of SiH SILOXANE 1 having 0.1588 % SiH, sunflower oil and isododecane (IDD) fluid were charged into a 1L flask. The mixture was mixed to homogeneous, purged with nitrogen, and then heated to 50°C, followed by the addition of a catalytic amount of platinum (IV) solution. The mixture went to 79°C resulted from reaction exotherm. The mixture was heated to 120°C and kept at 120°C for 5 hrs. The copolymer reaction mixture contained 0.0160% by weight of residual SiH (i.e. 69.97% of the SiH reacted with natural oil to form copolymer). To render the silicone-sunflower oil copolymer free of SiH, a calculated amount of alpha olefin 8 (1-octene) was incorporated to the mixture and reacted at 120°C for ~1 hr. The final copolymer was tested to be free of SiH by FTIR method. The final copolymer comprises 31.8% sunflower oil, 64.2% silicone and 4.0% alpha olefin 8 end capper. The copolymer was 51.1% solids in IDD.

| **Silicone-Sunflower oil copolymer** | |
|---|---|
| **Example ID** | 7 |
| **A)organohydrogensiloxane** | **SiH SILOXANE 1** |
| **B) Natural oil** | **Sunflower Oil** |
| **Wt.% natural oil in copolymer** | 31.8 |
| % Copolymer in reaction mixture | 51.1 |
| Carrier fluid type | IDD |

| **Si-Naturel oil copolymer step** | |
|---|---|
| SiH SILOXANE 1, g | 100.79 |
| Sunflower oil, g | 49.88 |
| Isododecane,g | 150.26 |
| Pt catalyst | 5 ppm |

| **End-capping step** | |
|---|---|
| alpha olefin end blocker (1-octene; 23% Vi), g | 6.37 |
| Total Batch, g | 307.30 |
| Appearance of final reacted mixture | Clear, light yellowish fluid |
| **Wt.% SiH in the starting mixture** | **0.0533** |
| **Wt.% SiH at the end of stage I reaction (SiH with oil)** | **0.0160** |
| **Wt.% SiH in the final mixture (after alpha olefin 8 capped)** | **0.000** |
| % **of SiH reacted at the end of step I** | **70.0%** |
| **Relative extent of SiH reacted in the final mixture** | **100.0%** |

A neat silicone-sunflower copolymer was obtained by stripping off the IDD carrier fluid from the above reaction mixture. The Si-sunflower oil copolymer was a clear, light yellowish viscous fluid.

### Examples 8

### Silicone-grape seed oil copolymer

In this example, prescribed amounts of MeH CYCLICS, grape seed oil, and isododecane (IDD) fluid were charged into a 1L flask. The mixture was mixed to homogeneous, purged with nitrogen, and then heated to 65C, followed by the addition of a catalytic amount of platinum (IV) solution. The mixture went to 82°C resulted from reaction exotherm. The mixture was heated to 120°C and kept at 120°C for 3 hrs. The copolymer reaction mixture contained 0.0134 % by weight of residual SiH (i.e. 82.5 % of the SiH reacted with natural oil to form copolymer). To render the silicone-sunflower oil copolymer free of SiH, a calculated amount of alpha olefin 8 (1-octene) was incorporated to the mixture and reacted at 120°C for ~1 hr. The final copolymer was tested to be only 0.0020% (or 20ppm) of SiH left by FTIR method. Additional alpha olefin 8 may be added to render the copolymer completely free of SiH, if desired.

| **Silicone-grape seed oil copolymer from SiH silicone cyclics and grape seed oil** | |
|---|---|
| **Example ID** | 8 |
| **A)organohydrogensiloxane** | MeH CYCLICS |
| **B) Natural oil** | Grape seed Oil |
| **Wt.% natural oil in copolymer** | **88.3** |
| % Polymer conc. | 50.6 |
| Carrier fluid type | IDD |

| **Si-Naturel oil copolymer step** | |
|---|---|
| MeH CYCLICS, g | 13.87 |
| Grape seed oil, g | 136.23 |
| Isododecane, g | 150.43 |
| Pt catalyst | 5 ppm |

| **End-capping step** | |
|---|---|
| alpha olefin end blocker (1-octene), g | 4.16 |
| Total Batch, g | 304.69 |
| Appearance of final reacted mixture | Clear, homogeneous, bright yellow |
| **Wt.% SiH in the starting mixture** | **0.0764** |
| **Wt.% SiH at the end of stage I reaction** | **0.0107** |
| **Wt.% SiH in the final mixture (after alpha olefin 8 capped)** | **0.0020** |
| **Relative extent of SiH reacted at the end of step I** | **86.0%** |
| **Relative extent of SiH reacted in the final mixture** | **97.4%** |

A neat silicone-grape seed oil copolymer was obtained by stripping off the IDD carrier fluid from the above reaction mixture. The Si-grape seed oil copolymer was a clear, light yellowish viscous fluid.

### Example 9

### Silicone-Shea Butter copolymer

In this example, prescribed amounts of MeH CYCLICS, Shea Butter and isododecane (IDD) fluid were charged into a 1L flask. The mixture was mixed to homogeneous, purged with nitrogen, followed by the addition of a catalytic amount of platinum (IV) solution. The mixture was heated to 120°C and kept at 120°C for 6 hrs. The copolymer reaction mixture contained 0.0264 % by weight of residual SiH (i.e. 54 % of the SiH reacted with Shea Butter to form copolymer). To render the silicone-sunflower oil copolymer free of SiH, a calculated amount of alpha olefin 8 (1-octene) was incorporated to the mixture and reacted at 120°C for -1 hr. The final copolymer was tested to be only 0.0027% (or 27 ppm) of SiH left by FTIR method. Additional alpha olefin 8 may be added to render the copolymer completely free of SiH, if desired.

| **Silicone-Shea Butter copolymer** | |
|---|---|
| **Example ID** | **9** |
| **A)organohydrogensiloxane** | MeH CYCLICS |
| **B) Natural oil** | HY-4003 Shea Butter |
| **Wt.% natural oil in copolymer** | 87.2 |
| % Polymer conc. | 51.5 |
| Carrier fluid type | IDD |

| **Si-Naturel oil copolymer step** | |
|---|---|
| MeH CYCLICS, g | 10.39 |
| Shea Butter, g | 139.73 |
| Isododecane, g | 150.44 |
| Pt catalyst | 5 ppm, |

| **End-capping step** | |
|---|---|
| alpha olefin end blocker (1-octene), g | **10.20** |
| Total Batch, g | 310.76 |
| Appearance of final reacted mixture | White solid wax at RT: turned clear, light yellow liquid at elevated temp (> 40C) |
| **Wt.% SiH in the un-reacted mixture** | **0.0574** |
| **Wt.% SiH at the end of stage I reaction** | **0.0264** |
| **Wt.% SiH in the final mixture (after alpha olefin 8 capped)** | **0.0027** |
| **Relative extent of SiH reacted at the end of step I** | **54.0%** |
| **Relative extent of SiH reacted in the final mixture** | **95.3%** |

A neat silicone-Shea Butter copolymer was obtained by stripping off the IDD carrier fluid from the above reaction mixture. The Si-Shea Butter copolymer was a white soft solid.

### Examples 10

### Silicone-Rice bran oil copolymer

In this example, prescribed amounts of MeH CYCLICS, Rice Bran oil, and isododecane (IDD) fluid were charged into a 1L flask. The mixture was mixed to homogeneous, purged with nitrogen, heated to 65°C, followed by the addition of a catalytic amount of platinum (IV) solution. The mixture was heated to 120°C and kept at 120°C for 6 hrs. The copolymer reaction mixture contained 0.0648 % by weight of residual SiH (i.e. 52.2 % of the SiH reacted with Rice Bran oil to form copolymer). To render the silicone-sunflower oil copolymer free of SiH, a calculated amount of alpha olefin 8 (1-octene) was incorporated to the mixture and reacted at 120°C for 2 hrs. The final copolymer was tested to be only 0.0021% (or 21 ppm) of SiH left by FTIR method. Additional alpha olefin 8 may be added to render the copolymer completely free of SiH, if desired. The final copolymer comprises of 71.6% rice bran oil, 14.0% silicones and 14.4% alpha olefin 8 end-capper. The reaction mixture is made to 53.8% solids in IDD.

| **Silicone-rice bran oil copolymer** | |
|---|---|
| **Example ID** | **10** |
| **A)organohydrogensiloxane** | MeH CYCLICS |
| **B) Natural oil** | **Rice Bran Oil** |
| **Wt.% natural oil in copolymer** | 71.6 |
| % Polymer conc. | 53.8 |
| Carrier fluid type | IDD |

| **Si-Naturel oil copolymer step** | |
|---|---|
| MeH CYCLICS, g | 24.59 |
| Rice Bran oil , g | 125.48 |
| isododecane, g | 150.33 |
| Pt catalyst | 5 ppm |

| **Endcapping step** | |
|---|---|
| alpha olefin end blocker (1-octene), g | 25.16 |
| Total Batch, g | 325.56 |
| Appearance of final reacted mixture | Clear, slightly yellowish fluid |
| **Wt.% SiH in the starting mixture** | **0.1357** |
| **Wt.% SiH at the end of step 1 reaction** | **0.0648** |
| **Wt.% SiH in the final mixture** | **0.0021** |
| **Relative extent of SiH reacted at the end of step I** | **52.2%** |
| **Relative extent of SiH reacted in the final mixture** | **98.5%** |

A neat silicone-rice bran oil copolymer was obtained by stripping off the IDD carrier fluid from the above reaction mixture. The Si-rice bran oil copolymer was a clear viscous fluid with slightly yellowish appearance.

### Example 11

### Silicone-almond oil copolymer derived from linear silicone and sunflower oil

In this example, prescribed amounts of SiH SILOXANE 1 (0.1588 % SiH), almond oil and isododecane (IDD) fluid were charged into a 1 L flask. The mixture was mixed to homogeneous, purged with nitrogen, and then heated to 65°C, followed by the addition of a catalytic amount of platinum (IV) solution. The mixture went to 80°C resulted from reaction exotherm. The mixture was heated to 120°C and kept at 120°C for 6 hrs. The copolymer reaction mixture contained 0.0253% by weight of residual SiH (i.e. 47.6% of the SiH reacted with natural oil to form copolymer). To render the silicone-sunflower oil copolymer free of SiH, a calculated amount of alpha olefin 8 (1-octene) was incorporated to the mixture and reacted at 120°C for 2 hrs. The final copolymer was tested to be free of SiH by FTIR method. The final copolymer comprises of 36.8% sweet almond oil, 57.1% silicone and 6.1% alpha olefin 8 end capper. The copolymer was made to 51.5% solids in IDD.

| **Silicone-almond oil copolymer** | |
|---|---|
| **Example ID** | **11** |
| **A)organohydrogensiloxane** | SiH SILOXANE 1 |
| **B) Natural oil** | Sweet Almond Oil |
| **Wt.% natural oil in copolymer** | **36.8** |
| % Copolymer in reaction mixture | 51.5 |
| Carrier fluid type | IDD |

| **Si-Naturel oil copolymer step** | |
|---|---|
| SiH SILOXANE 1 (0.1588% by FTIR), g | 91.29 |
| Sweet Almond oil, expeller pressed, g | 58.76 |
| Isododecane, g | 149.99 |
| Pt catalyst | 5 ppm |

| **Endcapping step** | |
|---|---|
| alpha olefin end blocker (1-octene), g | 9.79 |
| Total Batch, g | 309.83 |
| Appearance of final reacted mixture | Clear, homogeneous slightly yellowish |
| **Wt.% SiH in the un-reacted mixture** | **0.0483** |
| **Wt.% SiH at the end of step I reaction** | **0.0253** |
| **Wt.% SiH in the final mixture (after alpha olefin 8 capped)** | **0.0000** |
| **Relative extend of SiH reacted at the end of step I** | **47.6%** |
| **Relative extent of SiH reacted in the final mixture** | **100.00%** |

A neat silicone-almond oil copolymer was obtained by stripping off the IDD carrier fluid from the above reaction mixture. The Si-almond oil copolymer was a clear, light yellowish viscous fluid.

### Example 12

### Silicone-castor oil copolymer capped with allyl glycerol

In the example below, prescribed amounts of SiH SILOXANE 2 (0.35423% SiH), castor oil and isododecane (IDD) fluid were charged into a 1L flask. The initial mixture was cloudy, opaque and not homogeneous, purged with nitrogen, and then heated to about 85°C which the mixture turned clear and homogeneous, followed by the addition of a catalytic amount of platinum (IV) solution. The mixture was heated to 120°C and kept at 120°C for 6 hrs. The copolymer reaction mixture contained 0.0454% by weight of residual SiH (i.e. 40.3% of the SiH reacted with natural oil to form copolymer). To render the silicone-sunflower oil copolymer free of SiH, a calculated amount of allyl glycerol was incorporated to the mixture and reacted at 120°C for 2 hrs. The final copolymer was tested to about 0.0039% (39 ppm) of SiH remained in the mixture by FTIR method. Additional allyl glycerol may be added to render the final copolymer free of residual SiH. The final copolymer comprises of 56.6% castor oil, 35.1% silicone and 8.4% allyl glycerol end capper. The copolymer was made to 51.9% solids in IDD.

| **Silicone-castor oil copolymers** | |
|---|---|
| **Example ID** | **12** |
| **A)organohydrogensiloxane** | SiH SILOXANE 2 |
| **B) Natural oil** | Castor Oil |
| **Wt.% natural oil in copolymer** | 56.6 |
| % Polymer conc. | 51.9 |
| Carrier fluid type | IDD |

| **Si-Naturel oil copolymer step** | |
|---|---|
| SiH SILOXANE 2 (0.3543% SiH), g | 57.51 |
| Castor oil, 1^Press, g | 92.63 |
| Isododecane, g | 151.92 |
| Pt catalyst | 5 ppm |

| **End capping step** | |
|---|---|
| Allyl glycerol end blocker (26.47% Vi), g | 13.80 |
| Total Batch, g | 315.86 |
| Appearance of final reacted mixture | Milky mixture @ RT; turned clear > 50°C |
| **Wt.% SiH in the starting mixture** | **0.06783** |
| **Wt.% SiH at the end of step I reaction** | **0.0405** |
| **Wt.% SiH in final mixture (after allyl glycerol cap)** | **0.0039** |
| **Extent of SiH reacted at the end of step I** | **40.3%** |
| **Extent of SiH reacted in the final mixture** | **94.3%** |

### Example 13

### silicone-regetable oil elastomers via silicone-vegetable oil copolymers

The following elastomer gels were prepared using silicone-vegetable oil copolymers previously prepared and Si DIENE. The hydrosilylation reaction was successfully carried out in either pure IDD solvent or mixture of IDD and vegetable oil and elastomer gels were formed at a reaction temperature of 70°C.

| **Silicone-vegetable oil elastomers using copolymers** | | | | |
|---|---|---|---|---|
| **Example #** | **7A** | **7B** | **7C** | **7D** |
| **Component A): SiH containing Si-Natural copolymer** | **Example 4B** | **Example 4B** | **Example 4A** | **Example 4A** |
| **Component D: crosslinker type** | Si DIENE | Si DIENE | Si DIENE | Si DIENE |
| **Wt.% natural oil in gel** | 41.3 | 41.3 | 55.2 | 55.2 |
| Wt.% organic X-linker in gel | 21.5 | 21.5 | 15.4 | 15.4 |
| **Carrier fluid type** | **IDD** | **Vegetable oil / IDD** | **IDD** | **Vegetable oil / IDD** |
| SiH:Vi ratio | 1.00 | 1.00 | 1.00 | 1.00 |
| **% IEC in gel** | **20.0** | **20.0** | **20.0** | **20.0** |

| **Actuel amount** | | | | |
|---|---|---|---|---|
| Example 4B Vegetate oil copolymer, g | 10.97 | 11.03 | | |
| **Example 4A** /Vegetable oil copolymer, g | | | 14.34 | 14.36 |
| Si DIENE, g | 8.37 | 8.43 | 6.01 | 6.01 |
| Vegetable oil blend, g | | 20.00 | | 20.00 |
| IDD (Permethyl 99A), g | 60.72 | 40.70 | 59.60 | 39.80 |
| Syl-Off 4000, g | 0.05 | 0.08 | 0.05 | 0.08 |
| Total Batch, g | 69.14 | 69.21 | 80.00 | 80.25 |
| Gel appearance | Clear gel, very firm | Light yellowish firm gel | Clear firm gel | Light yellowish, soft gel |
| Process History | Gelled within 30 minutes into 70C | Gelled about 30 minutes | Gelled within 30 minutes. | Gelled in about 2 hrs. |

### Example 14

### silicone-shea butter elastomers via silicone-shea butter copolymers

The following elastomer gels were prepared using silicone-shea butter copolymers previously prepared in Example 5A. The hydrosilylation reaction was successfully carried out in either pure IDD solvent or mixture of IDD and shea butter and elastomer gels were formed at a reaction temperature of 70°C.

| **Silicone-shea butter elastomers from copolymers** | | |
|---|---|---|
| **Example #** | **14A** | **14B** |
| **Component A): SiH containing Si-Natural copolymer type** | **Silicone copolymer from Example 5a** | **Silicone copolymer from Example 5a** |
| **Component D: crosslinker type** | Si DIENE | Si DIENE |
| **Wt.% natural oil in gel** | **50.1** | **50.1** |
| **Wt.% organics in gel** | **18.6** | **18.6** |
| **Carrier fluid type** | **IDD** | **Shea Butter / IDD** |
| SiH:Vi ratio | 1.00 | 1.00 |
| **% IEC in gel** | **20.0** | **20.0** |

| **Actual amount** | | |
|---|---|---|
| **Silicone copolymer from Example 5a, g (50% in IDD)** | 17.62 | 17.62 |
| Si DENE. g | 7.19 | 7.21 |
| Shea Butter, g | | 20.02 |
| IDD (Permethyl 99A), g | 55.20 | 36.48 |
| Syl-Off 4000, g | 0.05 | 0.05 |
| Total Batch, g | 80.06 | 81.37 |
| Gel appearance at RT (overtime) | Clear, form gel (warm), with a few white particles suspended, after cool down | Light yellow, soft gel (warm): with lots of white particles suspended, after cool down. |
| Process History: note the gelation time | Gelled < 40 minutes | Gelled about 40 minutes |

### Example 15

### Silicone-Jojoba oil copolymer having residual SiH functionality

In the example below, prescribed amounts of MeH cyclics, jojoba oil and isododecane (IDD) fluid were charged into a 1L flask. The mixture was mixed to homogeneous, purged with nitrogen, and then heated to 50°C, followed by the addition of a catalytic amount of platinum (IV) solution. The mixture was heated to 120°C and kept at 120°C for 5 hrs. The copolymer reaction mixture contained 0.161 % by weight of residual SiH. The final copolymer comprises 86% jojoba oil and 14% silicone and contained 0.161 % SiH. The copolymer reaction was made to 70% solids in IDD.

| **Silicone-jojoba oil copolymer with residual SiH** | |
|---|---|
| **Example #** | **15** |
| **A)organohydrogensiloxane** | MeH CYCLICS |
| **B)Natural oil** | Jojoba oil |
| Wt.% Organics in copolymer | 86.0 |
| Carrier fluid type | IDD |
| SiH:Vi ratio | 0.80 |
| % Polymer conc. | 70.0 |

| **Si-jojoba reaction** | |
|---|---|
| MeH CYCLICS, g | 19.60 |
| Jojoba oil, g (Lipovol J) | 120.44 |
| Isododecane, g | 60.09 |
| Syl-Off 4000, g (0.5% Pt) | 0.25 |
| Total Batch, g | 200.38 |
| Appearance of reacted mixture | Moderate yellow, slightly hazy liquid |
| Wt.% SiH in the final mixture | 0.1614 |

### Example 16

### silicone-jojoba oil elastomers via silicone-jojoba oil copolymers

The following elastomer gels were prepared using silicone-jojoba oil copolymers previously prepared and Si DIENE. The hydrosilylation reaction was successfully carried out in either pure IDD solvent. The mixture of IDD and jojoba oil did not form elastomer gel at 70C and formed very soft gel at a reaction temperature of 100°C.

| **Silicone-,jojoba oil elastomers from copolymers** | | |
|---|---|---|
| **Example #** | **16A** | **16B** |
| **Component a): SiH containing Si-Natural copolymer type** | **Silicone copolymer of Example 15** | **Silicone copolymer of Example 15** |
| **Component D: crosslinker type** | Si DIENE | Si DIENE |
| **Wt.% Natural oil in gel** | **29.0** | **37.2** |
| Wt.% Organics in gel | 27.4 | 23.4 |
| **Carrier fluid type** | **IDD** | **Jojoba oil / IDD** |
| SiH:Vi ratio | 1.00 | 1.50 |
| **% IEC in gel** | **20.0** | **20.0** |

| **Actual amount** | | |
|---|---|---|
| **Example 15 Silicone copolymer, g** | 7.71 | 9.89 |
| Si DIENE. g | 10.65 | 9.18 |
| Jojoba oil, g | | 20.02 |
| IDD (Permethyl 99A), g | 61.81 | 41.72 |
| Syl-Off 4000, g (7 drops) | 0.05 | 0.05 |
| Total Batch, g | 80.21 | 80.86 |
| Gel appearance | Clear gel, very finn | Very soft, yellowish gel-like, lots of bubbles |
| Process History | Gelled within 30 minutes in 70C; held at 70C for 16 hrs. | No gelling even after 4 hrs @ 70C, very soft gel after 16 hrs. Post-cured at 100 C for 8 hrs. |

### Example 16

### Si-natural oil elastomers via direct process

The following elastomer gels were prepared by incorporating all the required components together and mixed to homogeneous, then placed in a 70 C water bath. As seen, the hydrosilylation reaction was successfully carried out and elastomer gels were formed at a reaction temperature of 70°C.

| **Silicone-natural elastomers formed by one-step method** | | | |
|---|---|---|---|
| **Example #** | **16A** | **16B** | **16C** |
| **A) organohydrogen-siloxane** | SiH SILOXANE 3 | SiH SILOXANE 3 | SiH SILOXANE 3 |
| **B) Natural oil** | **Shea butter** | **Jojoba oil** | **Vegetable oil** |
| **Component D)** | **Polycerin DMUS-80 PO20** | **Polycerin EMUS-80 PO20** | **Polycerin DMUS-80 PO20** |
| **Wt.% Organics in gel** | **22.6** | **22.6** | **22.6** |
| **Carrier fluid type** | **IDD** | **IDD** | **IDD** |
| **SiH:Vi ratio** | **1.20** | **1.20** | **1.20** |
| **% IEC in gel** | **20.0** | **20.0** | **20.0** |
| **Wt. % Natural oil/butter in gel composition** | 25.0 | 24.9 | 25.1 |

| **Actual amout** | | | |
|---|---|---|---|
| SiH SILOXANE 3 in IDD (50% conc.). g | 24.78 | 24.78 | 24.78 |
| Polycerin DMUS-80 MP020. g | 3.64 | 3.66 | 3.70 |
| Shea butter, g | 20.09 | | |
| Jojoba oil, g | | 20.02 | |
| Vegetable oil blend, g | 0.00 | | 20.14 |
| Isododecane, g | 31.70 | 31.76 | 31.62 |
| Syl-Off 4000, g | 0.05 | 0.05 | 0.05 |
| Total Batch, g | 80.26 | 80.28 | 80.28 |
| Gel appearance | Clear, firm gel; slightly yellowish | Clear, finn gel; slightly yellowish | Clear, firm gel; slightly yellowish |
| Process History | Gelled < 30 minutes @ 70C | Gelled < 30 minutes @ 70C | Gelled < 30 minutes @ 70C |
| **Texture analyzer, force 1, g** | **116.0** | **148.0** | **41.9** |
| **Texture analyzer, force-time 1-2, g** | **642.3** | **806.8** | **237.4** |
| **Gel hardness (as compression strength), N/m2** | **8,979** | **11,455** | **3,243** |
| **Viscosity of gel, N.s/m2 or poise (dynes/cm2)** | **49,715** | **62,448** | **18,375** |

### Example 17

### Si-natural oil elastomer blends

The following silicone-natural oil elastomer blends (SOEB) were prepared using a Waring blender to grind the gel mass to desired gel particle size and paste-like consistency.

| **Example #** | **17A** | **17B** | **17C** |
|---|---|---|---|
| **SOEB description** | **SOEB with Shea Butter in IDD** | **SOEB with Jojoba oil in IDD** | **SOEB with vegetable oil blend in IDD** |
| **Wt. % natural oil in final SEB** | **16.2** | **16.6** | **17.4** |
| **Wt.% organics in gel network** | **23.0** | **23.0** | **23.0** |

| **Composition** | | | |
|---|---|---|---|
| **Si-natural oil gel** | **Example 16A** | **Example 16B** | **Example 16C** |
| **Amount Gel, g** | 78.54 | 63.19 | 63.24 |
| Isododecane (IDD), g | 42.41 | 31.89 | 27.19 |
| 1% TPP, g | 0.18 | 0.19 | 0.18 |
| VINYL SILOXANE # 2 | 0.21 | 0.17 | 0.18 |
| Total Batch, g | 121.34 | 95.44 | 90.78 |
| SEB appearance | Hazy gel-like paste, turned clear gel-like paste at > 45C (reversible) | clear, very slight yellow paste | clear, very slight yellow paste |

| **SEB property** | | | |
|---|---|---|---|
| **% FEC** | **13.0** | **13.3** | **14.0** |
| **Viscosity (cps)** | **281,174** | **243,915** | **389,379** |

## Claims

1. A composition comprising reaction products of;
A) an organohydrogenpolysiloxane having at least two silicon bonded hydrogen atoms,
B) a vegetable oil containing at least one aliphatic unsaturated bond,
C) a hydrosilylation catalyst, and
D) an optional crosslinking compound containing at least two terminal aliphatic unsaturated groups in its molecule,
F) an optional alpha olefin compound,
wherein the reaction products contain at least one silicone copolymer formed by the addition of the silicon bonded hydrogen atoms of the organohydrogenpolysiloxane across the aliphatic unsaturated bond in the vegetable oil.

2. The composition according to claim 1 wherein the organohydrogenpolysiloxane comprises
- a linear organohydrogenpolysiloxane of general formula HR¹₂SiO-[R¹2SiO]ₓSiR¹₂H, where R¹ is a monovalent hydrocarbon and x ≥ 1,
- (CH₃)₂HSiO[(CH₃)₂SiO]ₓSiH(CH₃)₂, where x ≥ 1 or
- a organohydrogencyclosiloxane, preferably having an average formula [(CH₃)HSiO]₉ where g is 3 to 8.

3. The composition according to claim 1 wherein the vegetable oil is jojoba oil, sesame oil, primrose oil, rapeseed oil, palm oil, shea butter, or mixtures thereof.

4. The composition according to claim 1 wherein the silicone copolymer comprises
(a) an organopolysiloxane containing at least one structural unit of the average formula R¹ₘ (E)SiO (3-m)/2 where;
R¹ is a monovalent hydrocarbon group,
E is a fatty ester group attached to silicon in the structural unit by at least one Si - C bond wherein the carbon atom of the bond is one of the carbon atoms originally present in the aliphatic unsaturated bond of the vegetable oil,
m may vary from 0 to 2,
(b) an organopolysiloxane containing at least one structural unit of the average formula R¹ₘ SiO_{(3-m)/2} (E') SiO(₃₋ₘ)_{/2} R¹ₓ where;
R¹ is a monovalent hydrocarbon group,
E' is a fatty ester group attached to silicon in the structural unit by at least one Si - C bond wherein the carbon atom of the bond is one of the carbon atoms originally present in the aliphatic unsaturated bond of the vegetable oil,
m may vary from 0 to 2, or
(c) a structural unit of the average formula R¹₂(E)SiO-[R¹₂SiO]ₓ-SiR¹₂R² where E is a fatty ester group attached to silicon in the structural unit by at least one Si - C bond wherein the carbon atom of the bond is one of the carbon atoms originally present in the aliphatic unsaturated bond of the vegetable oil,
R¹ is a monovalent hydrocarbon group,
R² is hydrogen or E.

5. The composition of claim 4 wherein the organopolysiloxane in (a) or (b) is an organohydrogensiloxane, preferably an organohydrogencyclosiloxane.

6. A process for preparing the composition according to claim 1 comprising: reacting
A) an organohydrogenpolysiloxane having at least two SiH units;
B) a vegetable oil containing at least one aliphatic unsaturated bond; and
C) a hydrosilylation catalyst;
in the presence of
E) an optional solvent.

7. The process according to claim 6 wherein the molar ratio of the aliphatic unsaturated bonds in the vegetable oil to the SiH units in the organohydrogenpolysiloxane is ≥ 1.

8. The process according to claim 6 wherein the reaction is conducted in the absence of organic compounds or polymers having terminal aliphatic unsaturated groups.

9. The process according to claim 6 wherein the organohydrogenpolysiloxane
- comprises a linear organohydrogenpolysiloxane of general formula
HR¹₂SiO-[R¹₂SiO]ₓ-SiR¹₂H,
where R¹ is a monovalent hydrocarbon and x ≥ 1, or
- is an organohydrogencyclosiloxane having at least two SiH units on a siloxane ring, or
- has an average formula [(CH₃)HSiO]_{g} where g is 3 to 8.

10. The process according to claim 6 further comprising in the reaction:
D) a crosslinking compound containing at least two terminal aliphatic unsaturated groups in its molecule.

11. A process for preparing the composition according to claim 1 comprising:
I) reacting
A) an organohydrogenpolysiloxane having an average formula [(CH₃)HSiO]₉, where g is 3 to 8;
B) a vegetable oil containing at least one aliphatic unsaturated bond; and
C) a hydrosilylation catalyst;
wherein the reaction product contains un-reacted SiH units,
II) further reacting the product of step I) with
D) a crosslinking compound containing at least two terminal aliphatic unsaturated groups in its molecule
in the presence of
E) an optional solvent.

12. The process according to any one of claims 6-11 further comprising further reacting the product with F) an alpha olefin compound.

13. A process for preparing a gel composition comprising;
I) shearing the composition according to claim 1,
II) mixing additional quantities of D) the solvent.

14. The gel composition prepared according to claim 13.

15. A personal care product comprising the composition of claim 1, or 14.

## Patentansprüche

1. Zusammensetzung enthaltend Reaktionsprodukte von
A) einem Organowasserstoffpolysiloxan mit wenigstens zwei siliciumgebundenen Wasserstoffatomen,
B) einem Pflanzenöl, das wenigstens eine aliphatisch ungesättigte Bindung enthält,
C) einem Hydrosilylierungskatalysator und
D) einer optionalen vernetzenden Verbindung, die wenigstens zwei endständige aliphatisch ungesättigte Gruppen in ihrem Molekül enthält,
F) einer optionalen α-Olefinverbindung,
wobei die Reaktionsprodukte wenigstens ein Siliconcopolymer enthalten, das durch die Addition der siliciumgebundenen Wasserstoffatome des Organowasserstoffpolysiloxans an die aliphatisch ungesättigte Bindung in dem Pflanzenöl gebildet wird.

2. Zusammensetzung nach Anspruch 1, wobei das Organowasserstoffpolysiloxan enthält:
- ein lineares Organowasserstoffpolysiloxan der allgemeinen Formel HR¹₂SiO-[R¹₂SiO]ₓ-SiR¹₂H, worin R¹ ein einbindiger Kohlenwasserstoff ist und x ≥ 1 ist,
- (CH₃)₂HSiO[(CH₃)₂SiO]ₓSiH(CH₃)₂, worin x 2 1 ist, oder
- ein Organowasserstoffcyclosiloxan, vorzugsweise mit der mittleren Formel [(CH₃)HSiO]_{g}, worin g gleich 3 bis 8 ist.

3. Zusammensetzung nach Anspruch 1, wobei das Pflanzenöl Jojobaöl, Sesamöl, Nachtkerzenöl, Rapsöl, Palmöl, Sheabutter oder eine Mischung davon ist.

4. Zusammensetzung nach Anspruch 1, wobei das Siliconcopolymer enthält:
(a) ein Organopolysiloxan mit wenigstens einer Struktureinheit der mittleren Formel R¹ₘ(E)SiO(₃₋ₘ)_{/2}, worin
R¹ eine einbindige Kohlenwasserstoffgruppe ist,
E eine Fettsäureestergruppe ist, die an Silicium in der Struktureinheit über wenigstens eine Si-C-Bindung gebunden ist, wobei das Kohlenstoffatom der Bindung eines der Kohlenstoffatome ist, die ursprünglich in der aliphatisch ungesättigten Bindung des Pflanzenöls vorhanden waren,
m von 0 bis 2 variieren kann,
(b) ein Organopolysiloxan mit wenigstens einer Struktureinheit der mittleren Formel R¹ₘSiO_{(3-m)/2}(E')SiO_{(3-m)/2}R¹ₓ, worin
R¹ eine einbindige Kohlenwasserstoffgruppe ist,
E' eine Fettsäureestergruppe ist, die an ein Silicium in der Struktureinheit über wenigstens eine Si-C-Bindung gebunden ist, wobei das Kohlenstoffatom der Bindung eines der Kohlenstoffatome ist, die ursprünglich in der aliphatisch ungesättigten Bindung des Pflanzenöls vorhanden waren,
m von 0 bis 2 variieren kann oder
(c) eine Struktureinheit der mittleren Formel
R¹₂(E)SiO-[R¹₂SiO]ₓ-SiR¹₂R²,
worin E eine Fettsäureestergruppe ist, die an ein Silicium in der Struktureinheit über wenigstens eine Si-C-Bindung gebunden ist, wobei das Kohlenstoffatom der Bindung eines der Kohlenstoffatome ist, die ursprünglich in der aliphatisch ungesättigten Bindung des Pflanzenöls vorhanden waren,
R¹ eine einbindige Kohlenwasserstoffgruppe ist,
R² Wasserstoff oder E ist.

5. Zusammensetzung nach Anspruch 4, wobei das Organopolysiloxan in (a) oder (b) ein Organowasserstoffsiloxan, vorzugsweise ein Organowasserstoffcyclosiloxan, ist.

6. Verfahren zur Herstellung der Zusammensetzung nach Anspruch 1 umfassend Umsetzen von
A) einem Organowasserstoffpolysiloxan mit wenigstens zwei SiH-Einheiten,
B) einem Pflanzenöl, das wenigstens eine aliphatisch ungesättigte Bindung enthält, und
C) einem Hydrosilylierungskatalysator
in Gegenwart von
E) einem optionalen Lösungsmittel.

7. Verfahren nach Anspruch 6, wobei das molare Verhältnis der aliphatisch ungesättigten Bindungen in dem Pflanzenöl zu den SiH-Einheiten in dem Organowasserstofpolysiloxan ≥ 1 ist.

8. Verfahren nach Anspruch 6, wobei die Reaktion in Abwesenheit organischer Verbindungen oder Polymere mit endständigen aliphatisch ungesättigten Gruppen durchgeführt wird.

9. Verfahren nach Anspruch 6, wobei das Organowasserstoffpolysiloxan
- ein lineares Organowasserstoffpolysiloxan der allgemeinen Formel
HR¹₂SiO-[R¹₂SiO]ₓ-SiR¹₂H
enthält,
worin R¹ ein einbindiger Kohlenwasserstoff ist und x ≥ 1 ist, oder
- ein Organowasserstoffcyclosiloxan mit wenigstens zwei SiH-Einheiten an einem Siloxanring ist oder
- eine mittlere Formel [(CH₃)HSiO]_{g} aufweist, worin g gleich 3 bis 8 ist.

10. Verfahren nach Anspruch 6, das weiterhin in der Reaktion aufweist:
D) eine vernetzende Verbindung, die wenigstens zwei endständige aliphatisch ungesättigte Gruppen in ihrem Molekül enthält.

11. Verfahren zur Herstellung der Zusammensetzung nach Anspruch 1 umfassend:
I) Umsetzen von
A) einem Organowasserstoffpolysiloxan mit einer mittleren Formel [(CH₃)HSiO]_{g}, worin g gleich 3 bis 8 ist,
B) einem Pflanzenöl, das wenigstens eine aliphatisch ungesättigte Bindung aufweist, und
C) einem Hydrosilylierungskatalysator,
wobei das Reaktionsprodukt nichtumgesetzte SiH-Einheiten enthält,
II) weiter Umsetzen des Produkts aus Schritt I) mit
D) einer vernetzenden Verbindung, die wenigstens zwei endständige aliphatisch ungesättigte Gruppen in ihrem Molekül aufweist,
in Gegenwart von
E) einem optionalen Lösungsmittel.

12. Verfahren nach einem der Ansprüche 6-11, das weiterhin weiteres Umsetzen des Produkts mit F) einer α-Olefinverbindung umfasst.

13. Verfahren zur Herstellung einer Gelzusammensetzung umfassend:
I) Scheren der Zusammensetzung nach Anspruch 1,
II) Mischen zusätzlicher Mengen von D), dem Lösungsmittel.

14. Gelzusammensetzung herstellbar nach Anspruch 13.

15. Körperpflegeprodukt enthaltend die Zusammensetzung nach Anspruch 1 oder 14.

## Revendications

1. Composition comprenant des produits réactionnels de :
A) un organohydrogénopolysiloxane ayant au moins deux atomes d'hydrogène liés au silicium,
B) une huile végétale contenant au moins une liaison insaturée aliphatique,
C) un catalyseur d'hydrosilylation, et
D) un composé réticulant facultatif contenant au moins deux groupes insaturés aliphatiques terminaux dans sa molécule,
F) un composé d'alpha oléfine facultatif,
où les produits réactionnels contiennent au moins un copolymère de silicone formé par l'addition des atomes d'hydrogène liés au silicium de l'organohydrogénopolysiloxane à la liaison insaturée aliphatique dans l'huile végétale.

2. Composition selon la revendication 1 où l'organohydrogénopolysiloxane comprend
- un organohydrogénopolysiloxane linéaire de formule générale HR¹₂SiO-[R¹₂SiO]ₓ-SiR¹₂H, où R¹ est un hydrocarbure monovalent et x ≥ 1,
- (CH₃)₂HSiO[(CH₃)₂SiO]ₓSiH(CH₃)₂, où x ≥ 1 ou
- un organohydrogénocyclosiloxane ayant de préférence une formule moyenne [(CH₃)HSiO]_{g} où g est 3 à 8.

3. Composition selon la revendication 1 où l'huile végétale est l'huile de jojoba, l'huile de sésame, l'huile de primevère, l'huile de colza, l'huile de palme, le beurre de karité ou des mélanges de ceux-ci.

4. Composition selon la revendication 1 où le copolymère de silicone comprend
(a) un organopolysiloxane contenant au moins une unité structurale de formule moyenne R¹ₘ(E)SiO_{(3-m)/2} où :
R¹ est un groupe hydrocarboné monovalent,
E est un groupe ester gras lié au silicium dans l'unité structurale par au moins une liaison Si - C où l'atome de carbone de la liaison est l'un des atomes de carbone initialement présents dans la liaison insaturée aliphatique de l'huile végétale,
m peut varier de 0 à 2,
(b) un organopolysiloxane contenant au moins une unité structurale de formule moyenne R¹mSiO(₃₋ₘ)_{/2}(E')SiO(₃₋ₘ)_{/2}R¹ₓ où :
R¹ est un groupe hydrocarboné monovalent,
E' est un groupe ester gras lié au silicium dans l'unité structurale par au moins une liaison Si - C où l'atome de carbone de la liaison est l'un des atomes de carbone initialement présents dans la liaison insaturée aliphatique de l'huile végétale,
m peut varier de 0 à 2, ou
(c) une unité structurale de formule moyenne R¹₂(E)SiO-[R¹₂SiO]ₓ-SiR¹₂R² où E est un groupe ester gras lié au silicium dans l'unité structurale par au moins une liaison Si - C où l'atome de carbone de la liaison est l'un des atomes de carbone initialement présents dans la liaison insaturée aliphatique de l'huile végétale,
R¹ est un groupe hydrocarboné monovalent,
R² est l'hydrogène ou E.

5. Composition selon la revendication 4 où l'organopolysiloxane dans (a) ou (b) est un organohydrogénosiloxane, de préférence un organohydrogénocyclosiloxane.

6. Procédé pour préparer la composition selon la revendication 1 comprenant :
la réaction
A) d'un organohydrogénopolysiloxane ayant au moins deux unités SiH ;
B) d'une huile végétale contenant au moins une liaison insaturée aliphatique ;
et
C) d'un catalyseur d'hydrosilylation ;
en présence de
E) un solvant facultatif.

7. Procédé selon la revendication 6 où le rapport molaire des liaisons insaturées aliphatiques dans l'huile végétale aux unités SiH dans l'organohydrogénopolysiloxane est ≥ 1.

8. Procédé selon la revendication 6 où la réaction est conduite en l'absence de composés organiques ou de polymères ayant des groupes insaturés aliphatiques terminaux.

9. Procédé selon la revendication 6 où l'organohydrogénopolysiloxane
- comprend un organohydrogénopolysiloxane linéaire de formule générale HR¹₂SiO-[R¹₂SiO]ₓ-SiR¹₂H, où R¹ est un hydrocarbure monovalent et x ≥ 1, ou
- est un organohydrogénocyclosiloxane ayant au moins deux unités SiH sur un cycle siloxane, ou
- a une formule moyenne [(CH₃)HSiO]_{g} où g est 3 à 8.

10. Procédé selon la revendication 6 comprenant en outre dans la réaction :
D) un composé réticulant contenant au moins deux groupes insaturés aliphatiques terminaux dans sa molécule.

11. Procédé pour préparer la composition selon la revendication 1 comprenant :
I) la réaction
A) d'un organohydrogénopolysiloxane ayant une formule moyenne [(CH₃)HSiO]_{g} où g est 3 à 8 ;
B) d'une huile végétale contenant au moins une liaison insaturée aliphatique ; et
C) d'un catalyseur d'hydrosilylation ;
où le produit réactionnel contient des unités SiH qui n'ont pas réagi,
II) la réaction supplémentaire du produit de l'étape I) avec
D) un composé réticulant contenant au moins deux groupes insaturés aliphatiques terminaux dans sa molécule
en présence de
E) un solvant facultatif.

12. Procédé selon l'une quelconque des revendications 6-11 comprenant en outre la réaction supplémentaire du produit avec F) un composé d'alpha oléfine.

13. Procédé pour préparer une composition de gel comprenant :
I) le cisaillement de la composition selon la revendication 1,
II) le mélange de quantités supplémentaires de D) le solvant.

14. Composition de gel préparée selon la revendication 13.

15. Produit pour les soins personnels comprenant la composition selon la revendication 1 ou 14.
